# EUROPEAN PATENT APPLICATION

(11) **EP 4 501 342 A1**
(43) Date of publication of application: **05.02.2025**
(21) Application number: 24192124.6
(22) Date of filing: 31.07.2024
(51) Int. Cl.: A61K 36/53, A61K 36/80, A61K 35/64, A61K 33/42, A23L 33/00, A61K 8/00, A61P 17/00, A61P 31/00, A61P 31/02, A61P 31/04, A61P 31/10, A61P 31/12, A61P 39/00, A61Q 19/00, A61K 9/00

(54) **COMPOSITION WITH ANTIVIRAL, ANTIBACTERIAL AND ANTIFUNGAL ACTIVITY**

(30) Priority: 01.08.2023 IT 202300016338; 07.03.2024 IT 202400005170
(71) Applicant: Elysium Cell Bio Ita Srl, 80145 Napoli (IT)
(72) Inventor: FERRUCCI, Veronica, 80134 NAPOLI (IT); DE ANTONELLIS, Pasqualino, 83100 AVELLINO (IT); ZOLLO, Massimo, 80131 NAPOLI (IT)
(74) Representative: Bianchetti & Minoja with Trevisan & Cuonzo IPS SRL

(57) **Abstract**

The present invention discloses a pharmaceutical, nutraceutical, cosmetic, disinfectant or sanitizer composition comprising long-chain sodium polyphosphate, Mullein extract, Thyme extract, and Propolis extract, and the use thereof in the prevention or treatment of bacterial, viral or fungal infections, or in the prevention or treatment of airways and skin inflammations, by systemic, nasal-spray and topical administration.

## Description

The present invention discloses a pharmaceutical, nutraceutical, cosmetic, disinfectant or sanitizer composition comprising long-chain sodium polyphosphate, Mullein extract, Thyme extract, and Propolis extract, and the use thereof in the prevention or treatment of bacterial, viral or fungal infections, or in the prevention or treatment of airways and skin inflammations, by systemic, nasal-spray and topical administration.

### Background of invention

Respiratory illnesses due to infectious diseases have re-emerged in unforeseeable patterns after the pandemic. Indeed, as of 6^{th} January 2024, the morbidity and mortality weekly report (MMWR) by the Centers for Disease Control and Prevention (CDC) (https://www.cdc.gov/ncird/surveillance/respiratory-illnesses/index.html) reported n.76.574 'coronavirus disease 2019' (COVID-19), n.106.331 influenza (FLU) and n.19.748 respiratory syncytial virus (RSV) reported cases, with n.202.626 co-infected patients. This epidemiological picture underpins that the microbial co-infections retain their crucial role also in post-pandemics era.

Severe Acute Respiratory Syndrome Coronavirus 2 (SARS-CoV-2), a pathogen positive-sense single-stranded RNA virus, is the etiopathological agent of the pandemic COVID-19 responsible for substantial morbidity and mortality worldwide. Respiratory droplets and aerosols are both causes of SARS-CoV-2 transmission with a median incubation period of 4.5 days before symptoms onsets. Mild-to moderate COVID-19 patients experience cough, fever, self-reported olfactory and taste disorders, and gut microbiome dysbiosis. In contrast, the severe illness begins 1 week after symptom onset with dyspnoea and proceeds with a progressive respiratory failure ('acute respiratory distress syndrome', ARDS), systemic hyperinflammation, and extrapulmonary disease (*e*.*g*., cardiac, kidney and liver injury, coagulopathy and shock). Furthermore, COVID-19 patients often present persistent symptoms after infection, referred to as `long COVID', occurring in at least 10% of mild and severe SARS-CoV-2-infected people worldwide.

The microbial co-infection from virus, bacteria and fungi exacerbates the difficulties of treatment and prognosis of COVID-19 patients. Indeed, the bacterial 'community-acquired pneumonia' (CAP) co-infection confers a greater risk of 'in-hospital mortality' as compared to the other risk factors, such as advanced age and comorbidities. In this regard, a shift in the prevalence of respiratory pathogens in CAP occurred during COVID-19 pandemic. *Staphylococcus aureus* is the most frequent Gram-positive pathogen affecting the respiratory tract found in SARS-CoV-2 co-infections [1, 2]. Furthermore, changes of intestinal microbiota have been also reported in COVID-19 patients that showed reduction in bacteria diversity with increased opportunistic pathogens, such as *Streptococcus, Rothia, Escherichia coli* and *Shigella* and reduction of beneficial symbionts [3]. Regarding the other classes of etiological agents, Influenza virus and RSV are predominantly found among those respiratory viruses contributing to the co-infection with SARS-CoV-2 [4-6], especially among children aged 0-5 years; While *Candida albicans* is the most common coinfect fungus in COVID-19 patients [7]. Altogether these results suggest that COVID-19 infection increases the host susceptibility to other pathogen co-infections, probably via inducing long-lasting changes in innate- and adaptive-immune functions in both adults and children.

COVID-19 disease is generally accomplished by immune dysregulation in SARS-CoV-2-infected host that is firstly characterized by 'immunosuppression' [8] and later followed by 'hyperinflammation' [9]. The hyperinflammatory status is mediated by pro-inflammatory cytokines *(i.e.,* 'cytokine storm'), triggered by inflammatory signaling activation (e.g., nuclear factor kappa-light-chain-enhancer of activated B cells, NF-kB) that are responsible for immune cells dysfunctionalities (*e*.*g*., lymphopenia), thus increasing the susceptibility to co-infections by other microorganisms, including viruses, bacteria, and fungi [10].

Of importance, a crucial role during the early phases of infection is made by the antiviral innate immune response, including complement systems, interferons (IFN), chemokines, and immune cells (*e*.*g*., macrophages) whose role mainly consist in limiting the viral propagation via modulating cytokine production and, generally 2-3 weeks later after contact with the virus, inducing the adaptive immune response [11]. Thus, a failure of innate immunity may result in an abnormal acquired immune host response that causes those critical COVID-19 conditions.

Therefore, two different therapeutic time windows must be considered in the clinical management of the immunopathological viral response. For the early phases of infection or also 'prophylaxis', immunostimulant agents could be helpful to enhance the innate immunity against microbial infections. In contrast, once the virus has overcome the initiation of infection and the 'active' viral replicative stage has started, the systemic treatments should be aimed at inhibiting the hyperinflammation.

To date, only vaccines have been the most effective prophylactic treatment against COVID-19 pandemic. Nevertheless, the existing anti-Spike antibodies show a weakly activity or inactiveness against the new sub-lineages of the Variants of Concern (VOC) Omicron, such as BA.1, BA.1.1, BA.2, BA.4 and BA.5, including the latest emergent XBB: EG.5.1 and XBB.2.3 [12]. Other COVID-19 therapeutics include antivirals, antithrombotic, therapies for respiratory failure, neutralizing antibodies, modulators of the renin-angiotensin-aldosterone system, vitamins, immunomodulatory agents (IMIDs, *e*.*g*., glucocorticoids and cytokine antagonists) and anti-inflammatory drugs (AIDs). Thus, the development of effective treatment and prophylactic strategies against SARS-CoV-2 variants still represent an unmet medical need.

In the medical management of patients affected by COVID-19, some valuable therapeutic strategies imply the use of AIDs, especially those non-steroidal AIDs (NSAIDs), to mitigate the ongoing excessive inflammatory response occurring after the early phases of the disease. Indeed, several observational studies have indicated that the anti-inflammatory therapy with NSAIDs (*e*.*g*., Aspirin, Ibuprofen, and Indometacin), and especially the use of selective COX-2 inhibitors (*e*.*g*., Celecoxib and Nimesulide), can be safely used among patients positive to SARS-CoV-2 [13] showing beneficial effectiveness for the outpatient treatment of early COVID-19 symptoms, thus giving a 'protection' against the progression of the disease towards a severe illness. Nevertheless, the therapy with NSAIDs should be avoided in those patients younger than 12 years and during pregnancy, due their possible multiple adverse effects, including gastrointestinal bleeding, cardiovascular, nephrotoxicity, especially for people older than 65 years [14].

Nutraceuticals are potential alternatives to NSAIDs for the management of various inflammatory diseases, including mild to moderate COVID-19. Among the plant-based nutraceuticals, polyphenols (which are grouped in four classes: phenolic acids, flavonoids, stilbenes and lignans) have received particular interest because of their key roles in a variety of biological activities, including anti-inflammatory and immunomodulatory functions [15]. These functionalities exerted by polyphenols are mainly due to modulation of NF-kB pathway that, in turn, affects inflammatory cytokines production and, therefore, immune cells activities [15, 16].

Propolis, a resinous complex formula generated by honeybees, has been reported with nutraceutical benefits due to its high amounts of polyphenols that are responsible for antibacterial, antiviral, anti-inflammatory, antioxidant and immunostimulant activities [17]. Indeed, propolis has been reported with antimicrobial function against human pathogenic viruses (*e*.*g*., Influenza FLU, Herpes simplex type-1 HSV-1, RSV-1, RSV-2, human coronaviruses SARS-CoV-2 and HCoV-OC43, human adenovirus type 5, human rhinovirus type 14) and bacteria (*e.g., Escherichia coli* and *Pseudomonas aeruginosa),* especially those belonging to Gram-positive class (including *Staphylococcus aureus*).

*Thymus vulgaris* L. (species, *Plantarum;* family, *Lamiaceae;* commonly known as Thymus or Thyme) is an herbaceous, perennial aromatic and medicinal plant, rich in bioactive compounds (including polyphenols) with anti-oxidant, antibacterial (*e.g*., against *Escherichia coli, Pseudomonas aeruginosa, Streptococcus salivarius, Streptococcus mutans, Streptococcus pyogenes* and *Staphylococcus aureus*), antifungal (*i.e*., *Candida albicans*), antiviral (HSV, SARS-CoV-2, human immunodeficiency virus HIV, and Influenza), and anti-inflammatory functions [18].

*Verbascum thapsus* L. (species, *Plantarum;* family: *Scrophulariaceae;* commonly known as Verbascum or Mullein), is a monocarpic and biennial herb, and their flowers are a source of a wide variety of chemical constituents, including polyphenols. *Verbascum thapsus* L. has been reported with antiviral efficiency (against pseudorabies virus, human coronavirus HCov-229E, HSV-1 [19] and influenza A, antibacterial activity (especially against Gram positive strain, including *Staphylococcus aureus*) with anti-inflammatory properties.

Here, we have developed a novel nutraceutical formula by combining propolis, *Thymus vulgaris* L. (Thyme) and *Verbascum thapsus* L. (Mullein) with the addition of long chain inorganic polyphosphates (polyPs). These latest are currently used as dietary additives (E452i, as approved by European Food Safety Authority [EFSA]) with low acute oral toxicity, absence of genotoxicity and carcinogenicity, and with an acceptable daily intake (ADI) for phosphates expressed as phosphorus of 40 mg/kg of body weight per day [20]. Inorganic polyPs are compounds comprised of chains of five to many hundreds of inorganic phosphate (Pi) residues that modulate a variety of biological processes, including cell metabolism, inflammation [21], chaperone-like functions , and neural transmission. Recently polyPs have been described with an antimicrobial activity as new therapeutics for chronic wounds in humans due to their properties to entrap bacteria [22]. Furthermore, polyPs were also found to act as cytoprotective agents against HIV-1 [23] and SARS-CoV-2 [24-28]. Indeed, prophylactic, and therapeutic treatment with polyPs resulted in inhibition of SARS-CoV-2 active replication *in vitro.* Their mechanisms of action involve (*i*) binding to Angiotensin Converting Enzyme 2 (ACE2) on the host cells and to viral RNA-dependent RNA polymerase (RdRp) in infected cells promoting their proteasome-mediated degradation, *(ii)* impairment of viral-host cell interaction through their binding to viral Spike (S) protein, and *(iii)* inhibition of inflammatory cytokines belonging to the cytokine storm via NF-kB modulation [24, 25]. Furthermore, the delivery of polyPs with a non-ambulatory nebulizer system also resulted in antiviral effects *in vitro* against SARS-CoV-2 [24].

### Description of the invention:

The present invention provides a pharmaceutical, nutraceutical, cosmetic or disinfectant composition comprising long-chain sodium polyphosphate consisting of 40 to 160 phosphate units, more preferably 80 to 120 phosphate units, Mullein extract, Thyme extract, and Propolis extract.

Preferably, the pharmaceutical, nutraceutical, cosmetic or disinfectant composition includes:
- from 0.01% to 3%, preferably from 0.05% to 2.5% of long chain sodium polyphosphate, consisting of 40 to 160 phosphate units, preferably from 80 to 120 phosphate units
- from 0.1% to 5%, preferably from 0.2 to 2% of Verbascum extract
- from 0.1% to 5%, preferably from 0.2% to 2% of Thyme extract
- from 0.05% to 5%, preferably from 0.2% to 2% of Propolis extract
where said percentages refer to the total weight of the composition.

In a preferred embodiment of the invention, the long-chain sodium polyphosphate is obtained through a process which comprises heating the sodium monophosphate (monobasic sodium phosphate monohydrate H4NaO5P) at 700°C for a period of 1 hour and subsequent cooling. Preferably, at the end of the melting at 700°C, the product is poured into a stainless-steel mortar to allow an initial rapid cooling at room temperature from 5 to 30 minutes (15 minutes) and, subsequently, the solidified product is frozen at -80°C for 3 hours. At the end of the freezing procedure, the product is mechanically triturated using a homogenizer for 5 minutes, thus generating long-chain sodium polyphosphate consisting of 40 to 160 phosphate units, preferably 80 to 120 phosphate units in powder form which it is then dissolved in sterile isotonic saline solution (0.8% sodium chloride) in a sterile environment.

The qualitative measurement of the length of the long-chain sodium polyphosphate is carried out by an electrophoretic run at 15 V, 25 milliamp, for 60 minutes in the running buffer and its subsequent staining with a solution consisting of 20% methanol, 1% glycerol, 0.1% o-toluidine blue for 15 minutes at room temperature.

Preferably, the qualitative-quantitative measurement of the length of the long-chain sodium polyphosphate is carried out through transform infrared spectroscopy of Fourier (FTIR) with attenuated total reflectance (ATR), i.e. FITR-ATR. This approach is an analysis method used to determine the structure of individual molecules and the composition of molecular mixtures that uses modulated energy in the infrared band that exploits the penetration of light into a solid and liquid sample to acquire data on its structure and its composition in real time. In detail, the ATR-FTIR spectrum of long chain polyphosphate under sterile conditions is measured using a VARIAN 600-IR ATR-FTIR spectrometer (calibrated Pharmacopoeia Europaea, Ph. Eur.). The spectrum is recorded as 60 individual scans from 550 cm-1 to 4000 cm-1 using a resolution of 4 cm⁻¹. The spectra recorded under these conditions show all the characteristic bands of long-chain sodium polyphosphate. Faint signals of bound water molecules (H2O) are visible at 1650 cm⁻¹. Impurities or non-specific bands are not detected.

In a preferred embodiment, the composition of invention contains water-dispersible, dry, oily or sterol lipid extracts of Propolis, Thyme and Verbascum.

The invention composition may also contain excipients or pharmaceutical vehicles suitable for oral use, for topical use, for systemic use, preferably diluents, binders, lubricants, disintegrating agents and/or coloring agents.

Preferably, the invention compositions in the form of a nanospray, aerosol, mouthwash or toothpaste.

A form suitable for oral administration comprises long chain sodium polyphosphate, Mullein extract, Thyme extract, Propolis extract (hereafter: `basic formula') sodium chloride and water, wherein the sodium chloride is preferably 0.5% to 3.5%, more preferably 0.8% to 3% w/vol, and optionally one or more of Aloe vera (Aloe barbadensis) extract, Calendula officinalis L., capolini extract, Collagen, Echinacea (Echinacea purpurea (L.) Moench) extract, Erisimo (Sisymbrium officinalis) extract, Erisimo (Hedge mustard) extract, Honey, Hyaluronic Acid, Mint (Mentha Piperita L) extract, N-Acetyleysteine, Panthenol, Pinus sibirica extract, Plantain extract, polydecene, potassium sorbate, potassium phosphate dibasic, potassium phosphate monobasic, Rosmarinus officinalis cincoliferum extract, seawater, sodium benzoate, strawberry, Tocopheryl Acetate (Vitamin E), Xylitol.

A form suitable for topical use includes cream, serum, cleanser, mousse, intimate cleanser, lip balm or lipstick.

A cream form may comprise the basic formula, water, and optionally one or more of
1,2 hexanediol, acetyl hexapeptide-37, allantoin, Aloe barbadensis extract, aminomethyl propanol, Arnica montana, beeswax, benzyl alcohol, Berberis vulgaris bark extract, betaine, bisabolol, Borago officinalis extract, butylene glycol, butylhydroxytoluene, Butyrospermum parkii butter (Karitè), Calendula officinalis extract, Cananga odorata flower oil (Ylang Ylang oil), candelilla wax, caprylyl glycol, carbomer (carbopol), cera alba, cetearyl alcohol, cetyl alcohol, cetyl palmitate, citic acid, Citrus aurantium amara flower oil, coco-caprylate, cocos nucifera fruit, coumarin, ethylhexylglycerin, Cucumis sativas (Cucumber) seed oil, dead sea salts, decyl oleate, diatomaceous earth, dicaprylyl carbonate, dimethicone, disodium EDTA, ethylhexyl stearate, ethylhexylglycerin, glycerin, glyceryl caprylate, glyceryl linolenate, glyceryl oleate, glyceryl stearate SE, Hamamelis virginiana leaf extract, Helianthus annuus seed oil, hydrogenated vegetable oil, Jojoba oil, lactic acid, lanolin, lecithin, linalool, Malva sylvestris extract, Olea europaea fruit oil, olive oil, ozokerite, panthenol, paraffinum liquidum, parfum, pentylene glycol, Persea gratisima oil, petrolatum, phenoxyethanol, polyethylene glycol, polyglyceryl-3 caprate, Polyglyceryl 6 distearate, polysorbate, potassium palmitoyl hydrolyzed wheat protein, potassium sorbate, Prunus amygdalus dulcis oil, Rosa canina (Rose hip) fruit oil, sodium benzoate, sodium gluconate, sodium hyaluronate, sodium hydroxide, sodium polyaerylate, sorbitol, steareth-2, steareth-21, stearyl alcohol, Styrax benzoin resin extract, sucrose polystearate, theobroma cacao butter, tocopheryl acetate, Triticum vulgare germ oil caprylic/capric triglyceride, Triticum vulgaris oil, urea, vitamin E, xanthan gum, zinc oxide,
A sunscreen cream may comprise the basic formula, water and optionally one or more of collagen, anti-wrinkle peptides, which chelate metals and self-renew the skin selected from acetyl hexapeptide-37, acetyl hexapeptide-8, alumina dehydroacetic acid, aloe barbadensis leaf juice, arnica (arnica montana), astaxanthin, benzyl alcohol, bht, butyrospermum parkii butter, butylene glycol, calendula officinalis flower extract, caprylic/capric triglyceride, caprylyl glycol, carthamus tinctorius seed oil, palmitoyl tripeptide 5, cera alba, cetearyl alcohol, cetyl alchol, chondrus crispus powder, ci 77891/titanium dioxide, citric acid, collagen, coumarin, diatomaceous earth, dicaprylyl carbonate, dimethicone, disodium edta, dodecane, ethylexyl stearate, ethylhexylglycerin, glyceryl caprylate, glyceryl linoleate, glyceryl linolenate, glyceryl oleate, hamamelis virginiana leaf extract, helianthus annuus seed oil, hyaluronic acid, lanolin, lecithin, linalool, ozokerite, panthenol, parfum, paraffinum liquidum, persea gratissima oil, phenoxyethanol, polyglyceryl-2-dipolydroxystearate, polyglyceryl-3 caprate, polyglyceryl-3 disostearate, potassium sorbate, propanediol, prunus amygdalus duleis oil, simmondsie chinensis seed oil, sodium benzoate, sodium hyaluronate, sodium polyacrylate, sorbitol, stearic acid, steareth-2, steareth-21, stearyl alcohol, tritium vulgare germ oil, xanthan gum, zinc oxide.

The invention composition can be in a form suitable for systemic use, preferably in the form of tablets, capsules, powders, granules, suspensions, syrups.

The invention composition is conveniently used in the prevention or treatment of a viral infection caused by SARS-CoV-2 virus, respiratory syncytial virus (RSV-A or RSV-B), Influenza virus (FLU-A, FLU-B, or FLU-C) or herpes simplex, alone or in combination with an antiviral.

The invention composition can be used in the prevention or treatment of a bacterial or fungal infection caused by Gram positive bacteria, in particular Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucilaginosa, Rothia dentocariosa and Micrococcus luteus; Gram negative, in particular Escherichia coli, Pseudomonas aeruginosa, Acinetobacter lwoffii and Neisseria flavescens, bacteria belonging to the 'ESKAPE' class (Enterococcus faecium, Staphylococcus aureus, Klebsiella pneumoniae, Acinetobacter baumannii, Pseudomonas aeruginosa, and Enterobacter species); or fungi, in particular Candida albicans, , alone or in combination with another pharmacological agent, especially an antibiotic; or in the prevention or treatment of a fungal infection alone or in combination with another pharmacological agent, especially an antifungal.

The invention composition is preferably used in the treatment or prevention of oral candidiasis, mucositis, pharyngitis, gingivitis, stomatitis, dental interventions (dental therapies) caused by bacterial or fungal infections in immunosuppressed subjects treated with radiotherapy and/or chemotherapy for head and neck tumors; or in the treatment or prevention of oral candidiasis, mucositis, pharyngitis, gingivitis, stomatitis, periodontitis, dental interventions (dental therapies) caused by bacterial or fungal infections in immunosuppressed subjects treated with radiotherapy and/or chemotherapy for head and neck tumors, skin and breast tumors in therapeutic association with another pharmacological agent, especially with an antibiotic or antifungal.

The invention composition can be used in the treatment or prevention of inflammation of the skin due to lesions of a benign nature, in particular dermatitis, atopic dermatitis, contact dermatitis, seborrheic dermatitis, nummular dermatitis, stasis dermatitis, dyshidrotic dermatitis, neurodermatitis, perioral dermatitis, psoriasis (vulgaris, plaque, guttate, inverse, pustular, eritrodermic, nail, psoriatic arthritis), acne, rhagades, fissures, cracks, ulcers, sores, bedsores (pressure ulcers), or malignant, in particular squamous cell carcinoma, basal cell carcinoma or basal cell carcinoma, and melanoma.

The invention composition can be used for prevention or treatment of bacterial, viral, fungal infection including surgery or post-surgery intervention in human and veterinary applications.

### Detailed description of the invention

PolyPs are currently used as dietary additives without presenting an appreciable risk to health showing an ADI for phosphates of 40 mg/kg of body weight per day [20]. Recently, medium *(i.e.,* polyP40) and long *(i.e.,* polyP120) chain length polyPs (in terms of Pi residues) have been reported with antiviral activity against SARS-CoV-2 [24, 25]. Furthermore, polyPs have received attention as possible therapeutics with some recent studies exploring their use in several formulations (including nebulizer [24] and hydrogel [22]) for their antiviral [24-28], antibacterial [22] and immunomodulatory [24] activities.

The aim of the present invention is to design a new mixture with antimicrobial potential. It has been found that by combining natural ingredients already known to contain bioactive compounds (i.e. polyphenols) with therapeutic benefits in terms of antiviral, antibacterial and/or antifungal properties, with a long-chain sodium polyphosphate consisting of 40 to 160 phosphate units, preferably from 80 to 120 phosphate units, particularly obtained through the process of the invention based on melting, cooling/ solidification, freezing, trituration and dissolution, a product with increased antiviral, antibacterial and antifungal properties is obtained.

The natural ingredients are Propolis (Propolis), Common Thyme (Thymus vulgaris) and Mullein (Verbascum thapsus), preferably in the quantities described here.

The pharmaceutical, nutraceutical, cosmetic or disinfectant composition of invention preferably comprises:
- from 0.01% to 3%, more preferably from 0.05% to 2,5% of long chain sodium polyphosphate consisting of 40 to 160 phosphate units, preferably from 80 to 120 phosphate units
- from 0.1% to 5%, more preferably from 0.2 to 2% of Verbascum extract
- from 0.1% to 5%, more preferably from 0.2% to 2% of Thyme extract
- from 0.05% to 5%, more preferably from 0.2% to 2% of Propolis extract
where said percentages refer to the total weight of the composition.

In a preferred embodiment of the invention, the composition is obtained through a process based on melting, cooling/solidification, freezing, trituration and dissolution, which comprises the following steps:
(i) melting sodium monophosphate at a temperature from 300°C to 750°, preferably 700°C, for a period of from 1 hr to 3 hr, preferably 1.5 hr;
(ii) cooling the product of step (i) at room temperature for a period of 5 min to 30, preferably 15 min, thereby obtaining a solid product;
(iii) freezing the product of (ii) at a temperature from -20°C to -80°C, preferably -60°C for a period of from 1 hr to 24 hr, preferably 3 hrs;
   and optionally
(iv) mechanically triturating the product of (iii) for a period of 5 to 30, preferably 15 minutes, thereby obtaining a product in powder form;
(v) dissolving the product of (iv) in water and sodium chloride, consisting of 0.7% to 3.5%, preferably 0.8% to 3% w/vol NaCl in water.

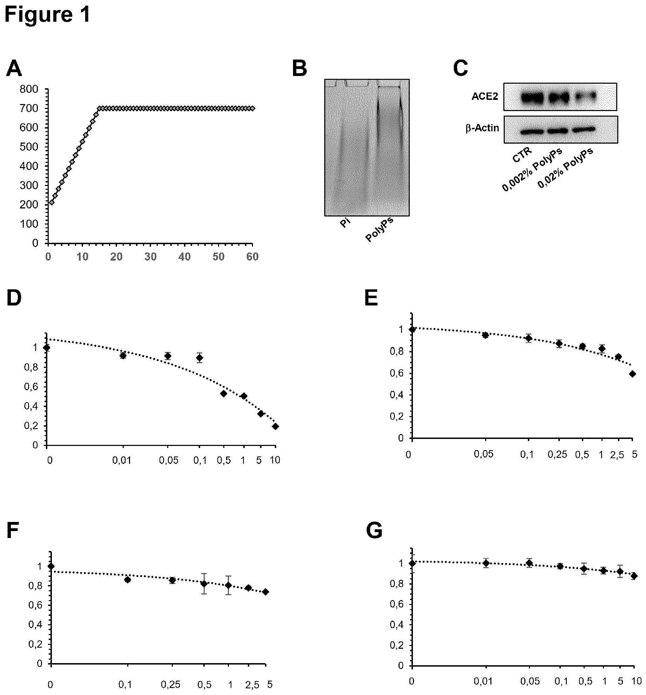

An example of thermal profile of long-chain sodium polyphosphate consisting of 40 to 160 phosphate units, preferably 80 to 120 phosphate units, obtained by melting monobasic sodium phosphate monohydrate H4NaO5P (≥ 99.0%, Carlo Erba, #480087) at 700°C (250gr in porcelain/aluminium crucible; LLF, #Gndie/Ca1469, 300 ml capacity) for one hour using an electric oven (Oven L 40/11 with Controller B 510; Nabertherm. com) is illustrated in **Figure** 1A. It can be seen that the time to reach 700°C is approximately 20 minutes (increase of 35°C/minute). Once 700°C is reached, the polymerization of the monobasic sodium phosphate monohydrate H4NaO5P in long-chain sodium polyphosphate consisting of 40 to 160 phosphate units, preferably 80 to 120 phosphate units, takes place at a constant temperature (700°C) for 1 hour.

Preferably, the long-chain sodium polyphosphate is measured qualitatively by staining with o-toluidine blue, according to the following procedure:
- Preparing an acrylamide gel containing: 7 M urea, 15-20% acrylamide, methylene bisacrylamide 1:20 and TBE (Tris-HCl Borate, EDTA pH: 8.0). To this 0.0625% APS and 0.0625% v/v TEMED are added for gel polymerization. The dimensions of the gel are as follows: length 30 cm, width 14 cm and thickness 0.75 cm.
- Preparing samples containing the generated long-chain sodium polyphosphate using a 2x loading 'buffer' containing: 0.2x TBE, 20% v/v glycerol, 0.1% bromophenol blue.
- running electrophoresis at constant power (15 W) until bromophenol blue migrates up to approx. 12cm. The current must not exceed 25 mA. The TBE is used as a running buffer. Pre-electrophoresis (300 V, 1 hour) can be performed to avoid poor band resolution due to excess salts.
- Staining of the gel with a solution consisting of 20% methanol, 1% glycerol, 0.1% o-toluidine blue for 15 minutes at room temperature.
- Decolorizing the gel with 20% v/v methanol, 1% v/v glycerol until the bands become visible.

An example of coloring for the qualitative measurement of the length of long-chain sodium polyphosphate synthesized through the process of the invention is illustrated in Figure 1B (unfused phosphate, Pi, was used as a control).

Preferably, the long-chain sodium polyphosphate is measured qualitatively-quantitatively through Fourier transform infrared spectroscopy (FTIR) with attenuated total reflectance (ATR), i.e. FITR-ATR. This approach is an analysis method used to determine the structure of individual molecules and the composition of molecular mixtures that uses modulated energy in the infrared band that exploits the penetration of light into a solid and liquid sample to acquire data on its structure and its composition in real time.

In detail, the ATR-FTIR spectrum of Long Chain Sodium Polyphosphate (consisting of 40 to 160 phosphate units, preferably 80 to 120 phosphate units, synthesized as described above) under sterile conditions is measured using an ATR-FTIR spectrometer FTIR VARIAN 600-IR (calibrated Pharmacopoeia Europaea, Ph. Eur.). The spectrum of Long Chain Sodium Polyphosphate (consisting of 40 to 160 phosphate units) is recorded as 60 individual scans from 550 cm-1 to 4000 cm-1 using a resolution of 4 cm-1. The spectra recorded under these conditions show all the characteristic bands of long-chain sodium polyphosphate. Faint signals of bound H20 are visible at 1650 cm-1. Impurities or non-specific bands are not detected.

The functionality of long-chain sodium polyphosphate can be determined by *in vitro* treatments on cells evaluating the ability of the aforementioned long-chain sodium polyphosphate to reduce the amount of the ACE2 protein. In this regard, long-chain sodium polyphosphate (approximately 120 phosphate units) has been reported with the ability to reduce the ACE2 protein by promoting its degradation mediated by the Proteasome [34]. An example of an in vitro assay for the evaluation of the functionality of the long chain sodium polyphosphate is illustrated in Figure 1C. In detail, Vero E6 cells (8 × 105 cells) were treated for 24 hours with 0.02 or 0.002% long-chain sodium polyphosphate consisting of 40 to 160 phosphate units synthesized through the fusion-based process of the invention, cooling/solidification, freezing, trituration and dissolution. Vero E6 cells treated for 24 hours with a 'vehicle' solution (i.e. 0.8% Sodium Chloride [NaCl]) were used as a negative control of the experiment. After 24 hours, the cells were lysed and the total proteins were used to carry out an immunoblotting assay to evaluate the functionality of long-chain sodium polyphosphate in inhibiting ACE2. The data in Figure 1C show a 30% and 20% reduction in the amount of ACE2 protein in Vero E6 cells treated with long-chain sodium polyphosphate at concentrations of 0.02 and 0.002%, respectively. Therefore, the invention composition is effective in promoting the degradation of the ACE2 protein.

The cytotoxicity of long-chain sodium polyphosphate was evaluated by MTS colorimetric assay (MTS tetrazolium salt: (3-(4,5-dimethylthiazol-2-yl)-5-(3-carboxymethoxyphenyl)-2-( 4-sulfophenyl)-2H-tetrazolium). For this purpose, human HEK-293T cells (1x105) were treated for 24 hours with increasing doses (from 0.01 to 10%) of long-chain polyphosphate. HEK-293T cells treated with a 'vehicle' solution (i.e. Chloride of Sodium [NaCl] at 0.8%) were used as a negative control of the experiment. After 24 hours, the treated cells were incubated for one hour with MTS tetrazolium salt, and the absorbance was measured at 490 nm. The data illustrated in Figure 1D shows that the IC50 inhibitory concentration of long-chain sodium polyphosphate in HEK-293T cells is 1.0405% (y = -0.1218x + 1.2086; R² = 0.9195). Therefore, the long-chain sodium polyphosphate does not show cytotoxicity in vitro.

The cytotoxicity of the individual components of the invention composition was evaluated by means of an MTS colorimetric assay. For this purpose, human HEK-293T cells (1×105) were treated for 24 hours with increasing doses of Propolis extracts (from 0.05 to 5%, Drawing 1E), Thyme vulgaris extract (from 0.05 to 5 %, Drawing 1F), and of Mullein (from 0.01 to 10%, Drawing 1G). Cells treated with the 'vehicle' solution (0.8% Sodium Chloride [NaCl]) are used as a negative control of the treatment. MTS assay data show the following IC50 inhibitory concentrations in HEK-293T cells: Propolis, 12.9083% (y = -0.0491x + 1.0649; R² = 0.8977; Figure 1E); Thymus vulgaris 42.5607% (y = -0.0359x + 0.9808; R² = 0.857; Figure 1F); Mullein, 38.3841% (y = -0.0181x + 1.0386; R² = 0.9153, Figure 1G).

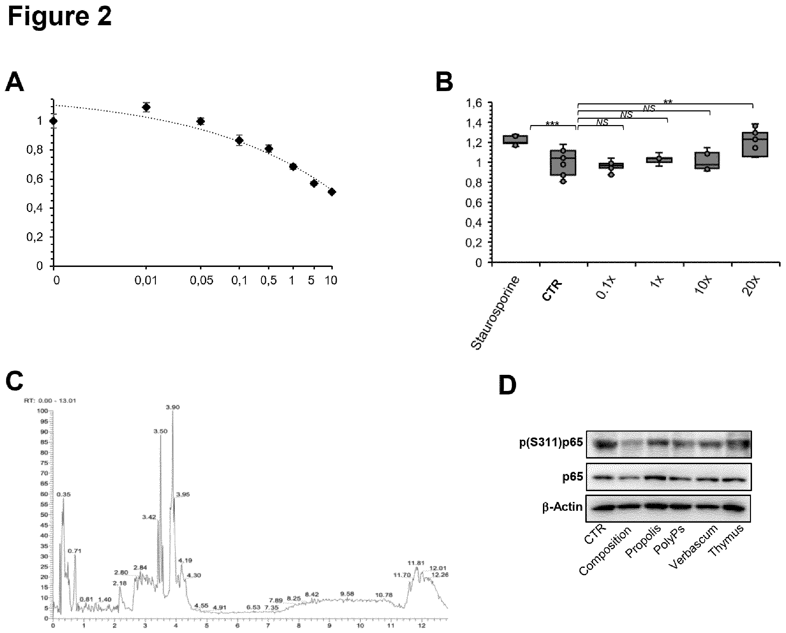

The cytotoxicity of the composition of the invention was also evaluated by MTS colorimetric assay. For this purpose, human HEK-293T cells (1×10⁵) were treated for 24 hours with increasing doses of the composition of the invention (from 0.1 to 10x). The IC50 for the composition of the invention in HEK-293T cells is 7.1023x (y = -0.0838x + 1.1938; R² = 0.9287), as illustrated in Figure 2A.

The data shows that the composition of the invention does not show cytotoxicity in vitro.

The absence of cytotoxicity of the composition object of the invention was further confirmed by evaluating the enzymatic activity of caspase 3 in human HEK-293T cells treated with different concentrations of the composition object of the invention (from 0.1 to 20x) for 24 hours. As a positive control, HEK-293T cells were stimulated with staurosporine (0.2 µM for 24 h). These data, illustrated in Figure 2B, show the induction of caspase 3 activity only at the highest dose of the pharmaceutical, nutraceutical, cosmetic or disinfectant composition object of the invention comprising long-chain sodium polyphosphate, Mullein extract (Verbascum Thapsus, or Mullein), common Thyme extract (Thymus vulgaris), Propolis extract (Propolis) tested here (i.e. 20x; Figure 2B).

In light of these results, the ingredients are preferably used at the following doses:
- 0.125% long-chain sodium polyphosphate
- 1.25% Mullein extract
- 1.25% Thyme extract
- 0.625% Propolis extract
- 0.8% Sodium Chloride Sodium Chloride [NaCl]
where said percentages refer to the total weight of the composition.

The composition containing long-chain sodium polyphosphate, dry extracs of Mullein, Thyme, and water dispersible Propolis at preferred doses was chemically profiled using ultra-high performance liquid chromatography (UHPLC) coupled to high resolution mass spectrometry (HRMS) using the quadrupole orbital ion trap analyzer (Q-Orbitrap; i.e. UHPLC-Q-Orbitrap HRMS [40, 50]) The data reported a high amount in polyphenols which corresponds to 1125.466 mg/g (i.e. 1.125 mg/ml), as illustrated in Figure 2C and Table 1). It is important to note that the quantity of polyphenols contained in the composition object of the invention is lower than the toxic dose and the 'NOAEL' dose (The no observed adverse effect level) reported in in vivo studies carried out on rats [51].

A chemical profile of the aqueous extracts of Thyme and Mullein was also performed. The extraction was carried out using "Naviglio extractor machine" (cod. serielab2L; Naviglio et al. JCHEM Educ.2015) a methodology conducted at room temperature for extraction in acqueus solution with a cycle constituted by a static and a dynamic phase; during the static phase, the liquid is put under pressure to about 8-9 atm with several passages in ON-OFF pressure until all the solid is extracted. Then we used the method above (UHPLC-Q-Orbitrap HRMS) to chemically profile the aqueous extracts in terms of Mullein, Thyme total polyphenol content (see Table 2).

These results suggest that the formulation of the invention, thanks to its high polyphenol content, may have immunomodulatory properties due to a synergistic action of the individual components. Therefore, the anti-inflammatory activity of the composition of the invention was measured through immunoblotting analysis carried out on human HEK-293T cells (1×10⁶) treated for 24 hours with the composition of the invention (0.01x), or with its individual components, i.e. long chain sodium polyphosphate synthesized through the process of the invention (0.125%), Propolis extract (0.625%), Thyme extract (1.25%) and Mullein extract (1.25%). HEK-293T cells treated with a 'vehicle' solution (0.8% Sodium Chloride [NaCl]) were used as a negative control of the experiment. The data show a synergistic activity of the components in inhibiting the inflammatory signaling pathway mediated by NF-kB. In detail, the immunoblotting data, illustrated in the 2D drawing, show a reduction of p65-NF-kB phosphorylated in Serine 311 by 70% in cells treated with the composition of the invention, by 30% in cells treated with the extract of Propolis, by 60% in cells with long-chain sodium polyphosphate, by 30% in cells treated with Mullein extract, and by 20% in cells treated with Thyme extract, compared to cells treated with vehicle solution.

Overall, these results indicate that the composition of the invention possesses benefits in terms of anti-inflammatory activity linked to the synergistic action of the individual components and to the content of polyphenols at non-cytotoxic doses, also suggesting an antimicrobial action against various pathogens, including those viral, bacterial and fungal diseases responsible for inflammatory infectious diseases.

To analyze the molecular targets and signaling pathways modulated by the composition of the invention RNA sequencing analysis (RNAseq) was performed in human cells. For this purpose, HEK-293T cells (1×10⁶) were treated with the composition of the invention at a non-toxic concentration (i.e. 0.01x) for 24 hours. HEK-293T cells (1×10⁶) treated with vehicle solution (0.8% Sodium Chloride [NaCl]) were used as a negative control for the experiments. RNAseq analysis showed n.1210 differentially expressed genes (DEG; Fold change of 2, p-value <0.05, Drawing 3A), of which n.732 and n.478 genes were up- or down-regulated, respectively, in cells treated with the composition of the invention.

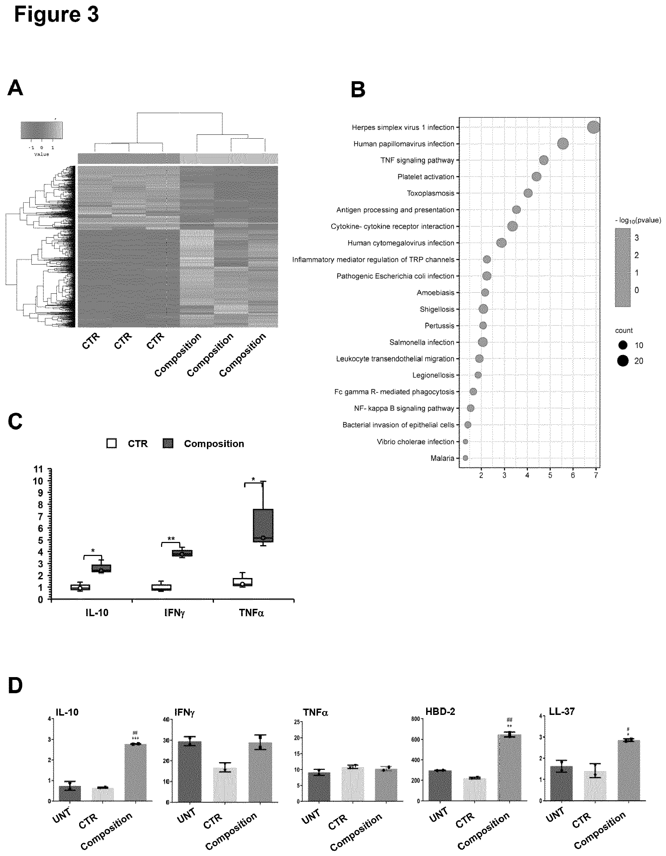

The analysis of dysregulated signaling pathways in HEK-293T cells (1×10⁶) treated with the composition of the invention at a non-toxic concentration (i.e. 0.01x) for 24 hours was performed using the Kyoto Encyclopedia of genes and genomes' (KEGG). The results indicate that the genes differentially expressed in HEK-293T cells treated with the composition of the invention are involved in pathologies caused by microbes, including infections by viruses (for example, HSV, papilloma, toxoplasma, cytomegalovirus) or bacteria (for example example, Escherichia coli, Legionella, Salmonella), as illustrated in Figure 3B. Furthermore, the results obtained also indicate that the genes differentially expressed in HEK-293T cells treated with the composition of the invention are involved in inflammatory processes, including tumor necrosis factor (TNF), cytokine-cytokine receptors and NF-kB signaling pathway, thus indicating that the composition of the invention can exert an immunomodulatory action (Figure 2B).

For all the above reasons, the potential modulation of inflammatory mediators involved in the innate immune response by the composition of the invention was studied via quantitative real-time PCR (qPCR) analysis. For this purpose, inflammatory genes mainly involved in the innate immune response were taken into consideration (e.g., IL-10, Interferon Gamma [IFNγ and Tumor Necrosis Factor alpha [TNFα]). Data obtained on HEK-293T cells treated with the composition of the invention at a non-toxic concentration of 0.01x for 24 hours show increased levels of inflammatory mediators involved in innate immunity IFNγ, TNFα and IL-10 in the cells HEK-293T, as shown in Figure 3C.

The results indicate that the pharmaceutical, nutraceutical, cosmetic or disinfectant composition of the invention causes an improvement in processes related to innate immunity through the stimulation of IFNy, TNFα and IL-10 in human HEK-293T cells.

To further validate these findings, we studied the modulation of the antimicrobial peptides human beta defensin 2 (HBD-2) and cathelicidin (LL-37) belonging to the innate immune response, together with their targets IFNγ, TNFα, IL-10 via enzyme immunoassay (Enzyme-Linked Immunosorbent Assay, 'ELISA') using the human enterocyte cell line Caco-2 (colon adenocarcinoma intestinal epithelial cells). For this purpose, Caco-2 cells were treated with the composition of the invention at a 0.01x concentration for 24 hours or with the vehicle solution (0.8% Sodium Chloride [NaCl]). Untreated Caco-2 cells were used as a further control of the experiment. Supernatants from Caco-2 cells treated as described above were used to measure beta-defensin 2 (HBD-2), LL-37, IL-10, IFNγ, and TNFα. The data obtained showed that the composition of the invention induces the release of IL-10, HBD-2 and LL-37, but not of TNFα and IFNγ, compared to cells treated with the vehicle solution or to untreated cells (negative controls of the experiment), as shown in Figure 3D. Due to the prominent role of HBD-2 and LL-37 in the innate immune response against bacterial, viral and fungal invasion [52], overall these data suggest that treatment with the composition of the invention can exert antimicrobial actions against several pathogens by modulating inflammatory processes.

The results indicate that the composition of the invention causes an improvement in processes related to innate immunity through the release of IL-10, HBD-2 and LL-37 in human Caco-2 cells.

Transcriptomic, qPCR and ELISA data indicate the modulation of inflammatory genes also involved in microbial infection. Therefore, we tested the potential antimicrobial activity of the composition of the invention against a variety of pathogens belonging to Gram positive or negative bacteria, and fungi. We also included in the tests the pathogens responsible for diseases of the oral cavity and upper respiratory tract that co-infect with SARS-CoV-2, and some belonging to the 'ESKAPE' family (e.g. Staphylococcus aureus and Pseudomonas aeruginosa). We used the method of the Clinical and Laboratory Standards Institute to test the effectiveness of the composition object of the invention using increasing concentrations (from 0.5 to 20x) against pathogens belonging to Gram-positive bacteria (Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucilaginosa, Rothia dentocariosa and Micrococcus luteus), Gram-negative bacteria (Escherichia coli, Pseudomonas aeruginosa, Acinetobacter lwoffii and Neisseria flavescens) and fungi (Candida albicans). The data show the bacteriostatic activity of the composition against all testicular bacteria and fungi with Minimum Inhibitory Concentration (MIC) values between 0.4x and 2x (with the exception of Enterococcus hirae which shows an MIC at a concentration of 4x), as reported in Table 3), and a Minimum Bactericidal Concentration (MBC), between 0.4 and 6. Furthermore, the antifungal action of the composition object of the invention has also been demonstrated with MIC and MBC values of 0.4x and 0.6x, respectively, against a clinical strain of Candida albicans. Overall, these data confirmed the antimicrobial activity of the composition of the invention which consists of bacteriostatic and bactericidal functions against Gram-positive and -negative bacteria, and Candida albicans.

Data shows that the pharmaceutical, nutraceutical, cosmetic or disinfectant composition of the invention can be used in the prevention or treatment of a bacterial or fungal infection caused by Gram positive bacteria (in particular Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucilaginosa, Rothia dentocariosa and Micrococcus luteus), Gram negative bacteria (in particular Escherichia coli, Pseudomonas aeruginosa, Acinetobacter lwoffii and Neisseria flavescens), bacteria belonging to the 'ESKAPE' class (Enterococcus faecium, Staphylococcus aureus, Klebsiella pneumoniae, Acinetobacter baumannii, Pseudomonas aeruginosa, and Enterobacter species), and fungi (in particular Candida albicans).

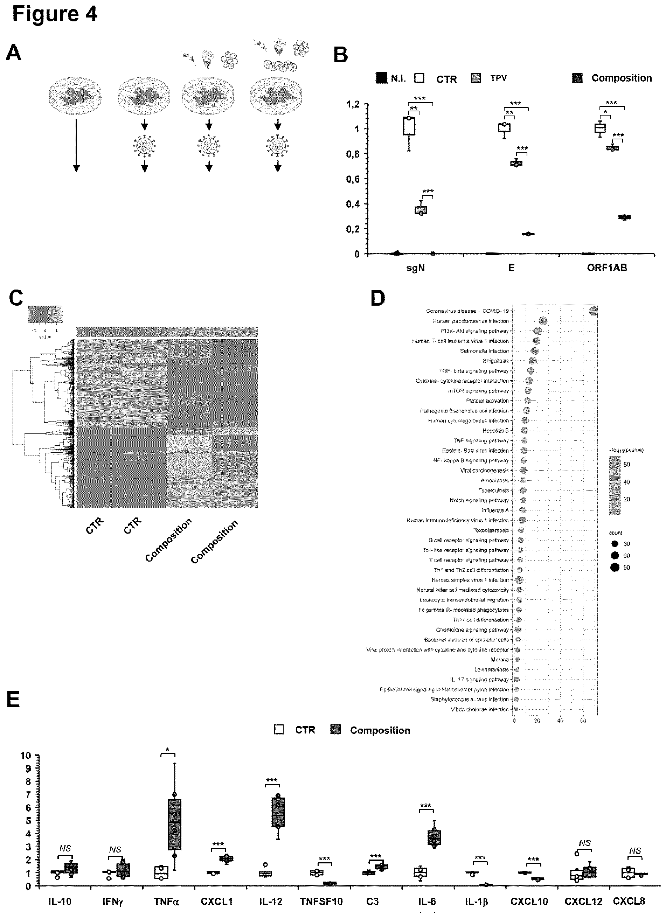

Since the composition modulates the expression of genes involved in the innate immune system, we also tested whether its "prophylactic" action could be extended to other clinically important viral pathogens. Therefore, we tested the antiviral efficacy of the composition of the invention against SARS-CoV-2, responsible for COVID-19. For this purpose, Caco-2 cells (5×10⁵) were treated with the composition of the invention or with a mixture of Mullein extract, Thyme extract, Propolis extract i.e. without long chain sodium polyphosphate. As a treatment control, Caco-2 cells were treated with the vehicle solution (0.8% Sodium Chloride [NaCl]). After 1 hour, pretreated Caco-2 cells were infected with SARS-CoV-2 virus (Omicron EG.5 variant, 3 MOI) for 48 hours, as illustrated in Figure 4A (i.e. prophylactic treatment). Uninfected cells were used as infection controls. The data obtained from real time quantitative PCR (qPCR) show a statistically significant greater decrease in SARS-CoV-2 viral genes (subgenomic sgN, Envelope, ORF1AB) in Caco-2 cells pre-treated with the composition of the invention compared to the same cells treated with the mixture of Mullein, Thyme, and Propolis extracts without long-chain sodium polyphosphate, as illustrated in Figure 4B. These results underline the preponderant role of long-chain sodium polyphosphate which acts synergistically with the other components of the composition of the invention, i.e. Mullein extract, Thyme extract and Propolis extract.

The immunomodulatory activity of the composition of the invention was confirmed in Caco-2 cells infected with SARS-CoV-2 through RNA sequencing analysis (RNAseq) and quantitative real time PCR (qPCR). In detail, Caco-2 cells were pre-treated with the composition of the invention (0.01x) or with vehicle solution (0.8% Sodium Chloride [NaCl]) and subsequently (after 1 hour), infected with SARS-CoV-2 (VOC Omicron EG.5, 3MOI) for 48 hours. RNAseq analysis performed on RNAs from Caco-2 cells treated as described above show no. 5353 genes differentially expressed ('Fold Change' of 2 and p-value <0.05) in cells infected by SARS-CoV-2 and pre-treated with the composition of the invention, as shown in Figure 4C. Furthermore, the results of the signaling pathway analysis carried out through the Kyoto Encyclopedia of Genes and Genomes (KEGG) using the differentially expressed genes ('Fold Change' of 2 and p-value <0.05) from the analyzes of RNA sequencing (RNAseq) performed in Caco-2 cells pre-treated with the composition of the invention at a concentration of 0.01x show deregulation of the signaling pathways involved in inflammation or pathologies caused by microbial infection, as illustrated in the Figure 4D. The immunomodulation exerted by the composition object of the invention on infected cells was further validated through the analysis of the mRNA expression of the inflammatory genes IL-10, IFNγ, TNFα, CXCL1, IL-12, TNFSF10, C3, IL- 6, IL-1β, CXCL10, CXCL12 and CXCL8 by real-time quantitative PCR (qPCR) analysis. The data presented in Figure 4E show that pre-treatment with the composition of the invention of Caco-2 cells infected with SARS-CoV-2 reduces the levels of TNFSF10, IL-1β, and CXCL10 (involved in hyper-inflammation and in the 'cytokine storm' and increases the expression levels of TNFα, CXCL1, IL-12, C3, and IL-6 (involved in the innate immune response).

These data indicate the antiviral efficacy of the composition of the invention in counteracting SARS-CoV-2 infection. Therefore, the composition of invention can be used in the prevention or treatment of a viral infection caused by SARS-CoV-2 in an individual who needs it. The composition of the invention can be administered alone or in therapeutic association with a further pharmacological agent, in particular with an antiviral agent for the prevention or treatment of a viral infection caused by SARS-CoV-2 in a subject who needs it.

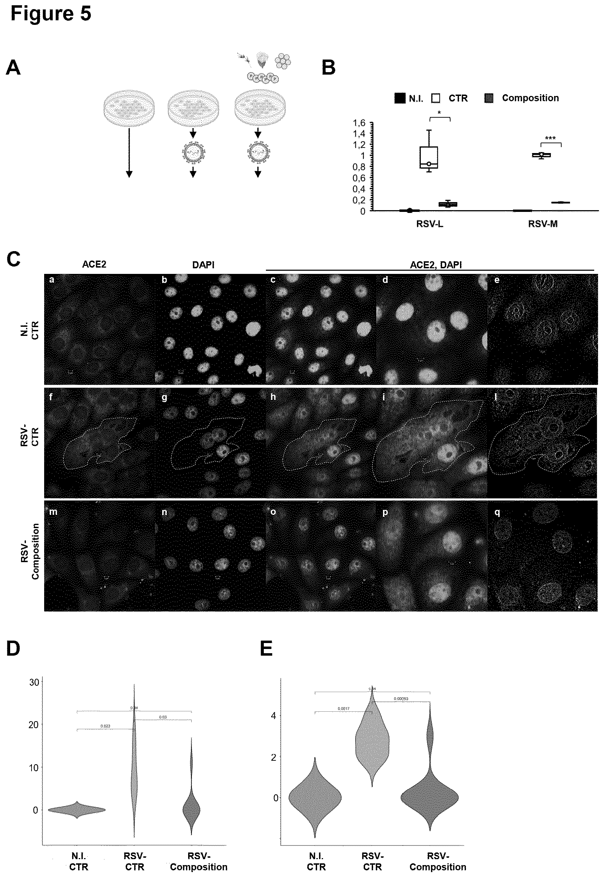

Due to the predominant role of RSV in co-infection with SARS-CoV-2 especially among children, we tested the potential antiviral activity of the composition of the invention against the respiratory syncytial virus RSV-A in vitro. For this purpose, Vero E6 cells (5×10⁵) were treated with the composition object of the invention (0.01x) or with the control vehicle solution, and, after 1 hour, they were infected with RSV-A virus for 72 hours, as illustrated in Figure 5A (i.e. prophylactic treatment). Uninfected cells were used as infection controls. The data obtained from real time quantitative PCR (qPCR) show a statistically significant decrease in the viral gene of the RNA dependent RNA polymerase (L) and the matrix (M) in those cells pre-treated with the composition of the invention and infected with RSV -A (Figure 5B). These data indicate the antiviral efficacy of the composition of the invention in counteracting RSV-A infection in vitro.

RSV infection begins with infected cells releasing new virions near healthy cells and then giving rise to cell fusion mechanisms that give rise to multinucleated cells called syncytia [53]. Therefore, we studied the ability of the composition of the invention to inhibit the propagation of RSV by reducing the formation of syncytia in infected cells. To this end, we performed immunofluorescence (IF) analysis on Vero E6 cells (using an anti-ACE2 antibody), to measure the number of fused cells in the syncytia following RSV-A infection. In detail, Vero E6 cells were treated with the composition of the invention (0.01x) or with the control vehicle solution, and, after 1 hour, they were infected with RSV-A virus for 72 hours, as illustrated in Figure 5A (i.e. prophylactic treatment). Uninfected cells were used as infection controls. Data show a mild syncytial phenotype in RSV-infected Vero E6 cells 72 hours into infection, with an average of 2 nuclei per syncytium (Figure 5C, panels f-l), compared to uninfected cells (Figure 5C , panels a-e). However, treatment with the composition of the invention strongly reduced the syncytia in Vero E6 cells infected by RSV-A (Figure 5C, panels m-q), resulting in a decrease in the percentage of syncytia (Figure 5D) and the number of nuclei per syncytium (Figure 5E). These data indicate the effectiveness of the composition of the invention in inhibiting the viral propagation of RSV while also influencing the formation of syncytia. Data shows that the composition of the invention can be used in the prevention or treatment of a viral infection caused by RSV in a subject who needs it. More in detail, the aforementioned composition of the invention can be administered alone or in therapeutic association with a further pharmacological agent, in particular with an antiviral agent for the prevention or treatment of a viral infection caused by RSV in a subject who suffers from it.

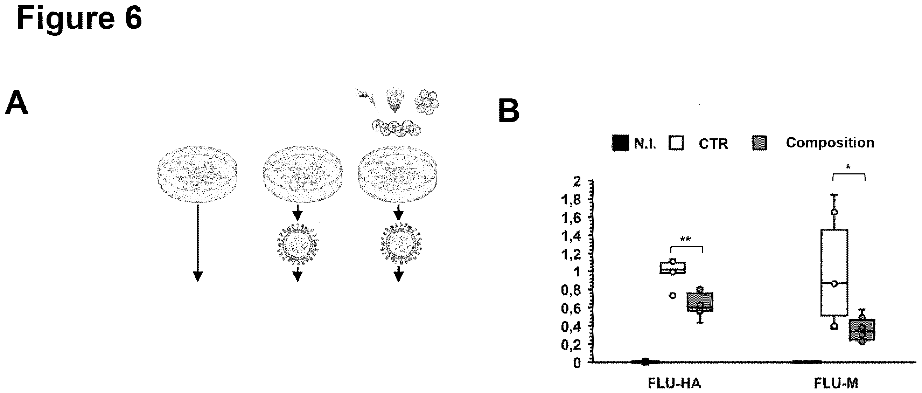

The antiviral activity of the composition of the invention was also tested against the influenza A virus in vitro. For this purpose, MDCK cells (isolated from normal kidney tissue of a cocker spaniel) were plated (1×10⁵ cells) and treated with the composition object of the invention at 0.01x concentration or with the vehicle (i.e. 0.8% Chloride of Sodium [NaCl]). After 1 hour, the cells were infected with viral particles belonging to Influenza A (FLU-A) at a concentration of 3 multiplicity of infection (MOI). After 6 h, cells were lysed for RNA extraction. Uninfected cells were used as a negative control for infection, as illustrated in Figure 6A. The data obtained from real time quantitative PCR (qPCR) show a statistically significant decrease in the viral hemagglutinin (HA) and matrix (M) gene in those cells pre-treated with the composition of the invention and infected with FLU-A (Figure 6B). These data indicate the antiviral efficacy of the composition of the invention in counteracting FLU-A infection in vitro. Data shows that the composition of the invention can be used in the prevention or treatment of a viral infection caused by influenza virus (FLU) in a subject who needs it. More in detail, the composition of the invention can be administered alone or in therapeutic association with a further pharmacological agent, in particular with an antiviral agent for the prevention or treatment of a viral infection caused by FLU influenza virus in a subject who needs it.

Overall, these results indicate the prophylactic efficacy of the composition of the invention during the early stages of infections against those bacterial and viral microbial pathogens commonly found in COVID-19 co-infections in the post-pandemic era, including viruses (RSV and FLU), gram positive and negative bacteria, and fungi (Candida Albicans).

In particular, the composition of the invention can be used in the prevention and treatment of an infection caused by:
- viruses, in particular from SARS-CoV-2 virus, respiratory syncytial virus (RSV-A or RSV-B), and Influenza virus (FLU-A, FLU-B or FLU-C)
- a bacterial or fungal infection caused by Gram positive bacteria (Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucilaginosa, Rothia dentocariosa and Micrococcus luteus), Gram negative (Escherichia coli, Pseudomonas aeruginosa, Acinetobacter lwoffii and Neisseria flavescens) and fungi (Candida albicans)
- in the treatment of skin inflammation due to lesions of a benign nature (e.g. in particular dermatitis (atopic dermatitis, contact dermatitis, seborrheic dermatitis, nummular dermatitis, stasis dermatitis, dyshidrotic dermatitis, neurodermatitis, perioral dermatitis, psoriasis (vulgaris, plaque, guttate,inverse, pustular, eritrodermic, nail, psoriatic arthritis), acne, rhagades, fissures, cracks, ulcers, sores, bedsores (pressure ulcers), or due to malignancy, in particular squamous cell carcinoma, cellular carcinoma basal or basal cell carcinoma, and melanoma)
- for sanitization and disinfection as a disinfectant to sanitize surfaces in healthcare and non-healthcare environments with a biocidal and oxidative effect.

For the envisaged therapeutical applications, the composition of invention is provided in a form suitable for oral use, preferably in the form of nanospray, aerosol, mouthwash or toothpaste; for topical use, preferably in the form of cream, serum, cleanser, mousse, intimate cleanser, lip balm or lipstick; or for systemic administration, preferably in the form of tablets, capsules, powders, granules, suspensions, syrups.

A composition suitable for oral use may include:
- Propolis extract
- Mullein extract
- thyme extract
- Long chain sodium polyphosphate consisting of 40 to 160 phosphate units, preferably 80 to 120 phosphate units
- sodium chloride, preferably 0.7% to 3.5%, more preferably 0.8% to 3% w/vol
- water
and, optionally, one of more of Aloe vera (Aloe barbadensis) extract, Calendula officinalis L., capolini extract, Collagen, Echinacea (Echinacea purpurea (L.) Moench) extract, Erisimo (Sisymbrium officinalis) extract, Erisimo (Hedge mustard) extract, Honey, Hyaluronic Acid, Mint (Mentha Piperita L) extract, N-Acetyleysteine, Panthenol, Pinus sibirica extract, Plantain extract, polydecene, potassium sorbate, potassium phosphate dibasic, potassium phosphate monobasic, Rosmarinus officinalis cincoliferum extract, seawater, sodium benzoate, strawberry, Tocopheryl Acetate (Vitamin E), Xylitol.

A composition in a form suitable for topical administration may include:
- Propolis extract
- Mullein extract
- thyme extract
- long chain sodium polyphosphate consisting of 40 to 160 phosphate units, preferably 80 to 120 phosphate units
- water
and one or more of 1, 2 hexanediol, acetyl hexapeptide-37, allantoin, Aloe barbadensis extract, aminomethyl propanol, Arnica montana, beeswax, benzyl alcohol, Berberis vulgaris bark extract, betaine, bisabolol, Borago officinalis extract, butylene glycol, butylhydroxytoluene, Butyrospermum parkii butter (Karitè), Calendula officinalis extract, Cananga odorata flower oil (Ylang Ylang oil), candelilla wax, caprylyl glycol, carbomer (carbopol), cera alba, cetearyl alcohol, cetyl alcohol, cetyl palmitate, citic acid, Citrus aurantium amara flower oil, coco-caprylate, cocos nucifera fruit, coumarin, ethylhexylglycerin, Cucumis sativas (Cucumber) seed oil, dead sea salts, decyl oleate, diatomaceous earth, dicaprylyl carbonate, dimethicone, disodium EDTA, ethylhexyl stearate, ethylhexylglycerin, glycerin, glyceryl caprylate, glyceryl linolenate, glyceryl oleate, glyceryl stearate SE, Hamamelis virginiana leaf extract, Helianthus annuus seed oil, hydrogenated vegetable oil, Jojoba oil, lactic acid, lanolin, lecithin, linalool, Malva sylvestris extract, Olea europaea fruit oil, olive oil, ozokerite, panthenol, paraffinum liquidum, parfum, pentylene glycol, Persea gratisima oil, petrolatum, phenoxyethanol, polyethylene glycol, polyglyceryl-3 caprate, Polyglyceryl 6 distearate, polysorbate, potassium palmitoyl hydrolyzed wheat protein, potassium sorbate, Prunus amygdalus dulcis oil, Rosa canina (Rose hip) fruit oil, sodium benzoate, sodium gluconate, sodium hyaluronate, sodium hydroxide, sodium polyaerylate, sorbitol, steareth-2, steareth-21, stearyl alcohol, Styrax benzoin resin extract, sucrose polystearate, theobroma cacao butter, tocopheryl acetate, Triticum vulgare germ oil caprylic/capric triglyceride, Triticum vulgaris oil, urea, vitamin E, xanthan gum, zinc oxide.

A composition in the form of sun protection cream may contain:
- Propolis extract
- Mullein extract
- thyme extract
- long chain sodium polyphosphate consisting of 40 to 160 phosphate units, preferably 80 to 120 phosphate units
- water
and one or more of collagen; anti-wrinkle peptides, which chelate metals and self-renew the skin, selected from acetyl hexapeptide-37, acetyl hexapeptide-8; alumina dehydroacetic acid, aloe barbadensis leaf juice, arnica (arnica montana), astaxanthin, benzyl alcohol, bht, butyrospermum parkii butter, butylene glycol, calendula officinalis flower extract, caprylic/capric triglyceride, caprylyl glycol, carthamus tinctorius seed oil, palmitoyl tripeptide 5, cera alba, cetearyl alcohol, cetyl alchol, chondrus crispus powder, ci 77891/titanium dioxide, citric acid, collagen, coumarin, diatomaceous earth, dicaprylyl carbonate, dimethicone, disodium edta, dodecane, ethylexyl stearate, ethylhexylglycerin, glyceryl caprylate, glyceryl linoleate, glyceryl linolenate, glyceryl oleate, hamamelis virginiana leaf extract, helianthus annuus seed oil, hyaluronic acid, lanolin, lecithin, linalool, ozokerite, panthenol, parfum, paraffinum liquidum, persea gratissima oil, phenoxyethanol, polyglyceryl-2-dipolydroxystearate, polyglyceryl-3 caprate, polyglyceryl-3 disostearate, potassium sorbate, propanediol, prunus amygdalus duleis oil, simmondsie chinensis seed oil, sodium benzoate, sodium hyaluronate, sodium polyacrylate, sorbitol, stearic acid, steareth-2, steareth-21, stearyl alcohol, tritium vulgare germ oil, xanthan gum, zinc oxide.

### Description of the Figures

**Figure 1: (A)** Thermal synthesis curve of long-chain sodium polyphosphate. In detail, long-chain polyphosphate was produced by a method based on the melting of monobasic sodium phosphate monohydrate H4NaO5P (≥ 99.0%, Carlo Erba, #480087) carried out at 700°C (250gr in porcelain/aluminium crucible; LLF, #Gndie/Ca1469, 300 ml capacity) using an electric oven (Oven L 40/11 with Controller B 510; Nabertherm.com) . The time to reach 700°C is approximately 20 minutes (increase of 35°C/minute). Once 700°C is reached, the melting system operates at a constant temperature which is recorded for 1 hour. X axis: time (min); Y axis: temperature (C°). **(B)** Staining of long-chain sodium polyphosphate synthesized by melting monobasic sodium monophosphate monohydrate (H4NaO5P) at 700°C for 1 hour , cooled/solidified at room temperature for 10 minutes, frozen at -80°C for 3 hours, mechanically triturated with a homogenizer for 5 minutes, and dissolved in sterile isotonic saline (0.8% sodium chloride) in a sterile environment. The qualitative measurement of the length of the long-chain sodium polyphosphate was carried out by an electrophoretic run (7 molar Urea, 15-20% acrylamide [1:20 methylene: bis acrylamide] in 1X Tris-HCl Borate EDTA pH: 8.0) at 15 V, 25 milliAmp, for 60 minutes in running buffer (i.e. 0.2X Tris-borate EDTA pH 8.0 [TBE], 20% glycerol, 0.1% bromophenol blue) and its subsequent staining with a solution consisting of 20% methanol, 1% glycerol, 0.1% o-toluidine blue for 15 minutes at room temperature. Unfused phosphate (Pi) was used as a control. Pi, unfused phosphate; polyPs, Long-chain sodium polyphosphate (consisting of 40 to 160 phosphate units, preferably 80 to 120 phosphate units). **(C)** Representative immunoblotting analysis (with antibodies against the indicated proteins: ACE2 and β-Actin) in Vero E6 cells (8 × 105 cells) treated for 24 hours with 0.02 or 0.002% long-chain sodium polyphosphate synthesized by melting monobasic sodium monophosphate monohydrate (H4NaO5P) at 700°C for 1 hour, cooled/solidified at room temperature for 10 minutes, frozen at -80°C for 3 hours, mechanically triturated with a homogenizer for 5 minutes, and dissolved in sterile isotonic saline solution (0.8% sodium chloride) in a sterile environment. Vero E6 cells treated for 24 hours with 'vehicle' solution (CTR, i.e. 0.8% Sodium Chloride [NaCl]) were used as a negative control of the experiment. Data show a 30% and 20% reduction in ACE2 in Vero E6 cells treated with 0.02% and 0.002%, respectively, long-chain sodium polyphosphate compared to cells treated with the vehicle solution. The densitometric analysis of the intensities of the ACE2 bands normalized on β-Actin was carried out in 3 independent experiments. polyPs, Long-chain sodium polyphosphate (consisting of 40 to 160 phosphate units, preferably 80 to 120 phosphate units). CTR, Vero E6 cells treated with the 'vehicle' solution (i.e. 0.8% Sodium Chloride [NaCl]).
   **(D)** Graph showing MTS colorimetric assay results (MTS tetrazolium salt: (3-(4,5-dimethylthiazol-2-yl)-5-(3-carboxymethoxyphenyl)-2-(4-sulfophenyl)-2H-tetrazolium) performed in HEK-293T cells (1×10⁵) treated for 24 hours at increasing doses (from 0.01 to 10%) with synthesized long-chain polyphosphate (consisting of 40 to 160 phosphate units, preferably 80 to 120 phosphate units) by melting monobasic sodium monophosphate monohydrate (H4NaO5P) at 700°C for 1 hour, cooling/solidifying it at room temperature for 15 minutes, freezing at -80°C for 3 hours, mechanical trituration with a homogenizer for 5 minutes, and dissolving in sterile isotonic saline (0.8% sodium chloride) in a sterile environment. HEK-293T cells treated with a vehicle solution (CTR, i.e. 0.8% sodium chloride [NaCl]. %) were used as a negative control of the experiment. The absorbance values measured at 490 nm are shown as 'fold' on cells treated with the 'vehicle' solution. Values represent the mean ± standard deviation (SD). The IC50 for Long Chain Sodium Polyphosphate in HEK-293T cells is 1.0405% (regression equation: y = -0, 1218x + 1.2086; IC50 was calculated by nonlinear regression analysis {[inhibitor] versus response (three parameters)} with 'Quest Graph^{™} IC50 Calculator' (AAT Bioquest, Inc., 8 January 2024; https://www.aatbio .com /tools/ic50-calculator. The graphs were generated with Microsoft Excel (version 16.82) phosphate units, preferably from 80 to 120 phosphate units) Y axis: absorbance values measured at 490 nm shown as 'fold' on cells treated with the 'vehicle' solution; %) of long-chain polyphosphate (consisting of 40 to 160 phosphate units, preferably 80 to 120 phosphate units). X axis: increasing doses (from 0.05 to 5%) of polyPs
   **(E)** Graph showing MTS colorimetric assay results (MTS tetrazolium salt: (3-(4,5-dimethylthiazol-2-yl)-5-(3-carboxymethoxyphenyl)-2-(4-sulfophenyl)-2H-tetrazolium) performed in HEK-293T cells (1×10⁵) treated for 24 hours at increasing doses of dry Propolis extract (from 0.05 to 5%). ] at 0.8%) were used as a negative control of the experiment. The absorbance values measured at 490 nm are shown as 'fold' on the cells treated with the 'vehicle' solution. The values represent the mean ± standard deviation. SD). , R2 and inhibitory concentration IC50 values. The IC50 was calculated by non-linear regression analysis {[inhibitor] versus response (three parameters)} with the 'Quest Graph^{™} IC50 Calculator' (AAT Bioquest, Inc., January 8, 2024; https://www.aatbio.com/tools/ic50-calculator. The graphs were generated with Microsoft Excel (version 16.82). N = 6 independent experiments per group. R2, coefficient of determination; CTR, cells treated with the 'vehicle' solution (0.8% Sodium Chloride [NaCl]). Y axis: absorbance values measured at 490 nm shown as 'fold' on cells treated with the 'vehicle' solution; X axis: increasing doses (from 0.05 to 5%) of propolis.
   **(F)** Graph showing MTS colorimetric assay results (MTS tetrazolium salt: (3-(4,5-dimethylthiazol-2-yl)-5-(3-carboxymethoxyphenyl)-2-(4-sulfophenyl)-2H-tetrazolium) performed in HEK-293T cells (1×10⁵) treated for 24 hours at increasing doses of Thyme vulgaris leaf extract (from 0.05 to 5%). HEK-293T cells treated with a 'vehicle' solution (i.e. Sodium Chloride [0.8% NaCl] were used as a negative control of the experiment. The absorbance values measured at 490 nm are shown as 'fold' on the cells treated with the 'vehicle' solution. The values represent the mean ± deviation standard (SD). The IC50 for Thymus extract in HEK-293T cells is 42.5607% (regression equation: y = -0.0359x + 0.9808; R² = 0.8571). Regression equation, R2 and inhibitory concentration IC50 values were calculated by non-linear regression analysis {[inhibitor] versus response (three parameters)} with the 'Quest Graph^{™} IC50 Calculator' (AAT Bioquest, Inc., January 8, 2024; https://www.aatbio.com/tools/ic50-calculator. The graphs were generated with Microsoft Excel (version 16.82). N = 6 independent experiments per group. R2, coefficient of determination; CTR, cells treated with the 'vehicle' solution (0.8% Sodium Chloride [NaCl]). Y axis: absorbance values measured at 490 nm shown as 'fold' on cells treated with the 'vehicle' solution; X axis: increasing doses (from 0.05 to 5%) of thymus.
   **(G)** Graph showing MTS colorimetric assay results (MTS tetrazolium salt: (3-(4,5-dimethylthiazol-2-yl)-5-(3-carboxymethoxyphenyl)-2-(4-sulfophenyl)-2H-tetrazolium) performed in HEK-293T cells (1x105) treated for 24 hours at increasing doses of Mullein extract 'verbascum thapsus' (from 0.01 to 10%). HEK-293T cells treated with a 'vehicle' solution (i.e Sodium [NaCl] 0.8%) were used as a negative control of the experiment. The absorbance values measured at 490 nm are shown as 'fold' on the cells treated with the vehicle solution. The values represent the mean ± standard deviation (SD). The IC50 for Mullein extract in HEK-293T cells is 38.3841% (regression equation: y = -0.0181x + 1.0386; R² = 0.9153). the regression equation, the R2 and inhibitory concentration IC50 values. The IC50 was calculated by non-linear regression analysis {[inhibitor] versus response (three parameters)} with the 'Quest Graph^{™} IC50 Calculator' (AAT Bioquest, Inc., January 8, 2024; https://www.aatbio.com/tools/ic50-calculator. The graphs were generated with Microsoft Excel (version 16.82). N = 6 independent experiments per group. R2, coefficient of determination. Y axis: absorbance values measured at 490 nm shown as 'fold' on cells treated with the 'vehicle' solution; X axis: increasing doses (from 0.01 to 10%) of mullein.
**Figure 2: (A)** Graph showing the results of the MTS (tetrazolium salt) colorimetric assay MTS: (3-(4,5-dimethylthiazol-2-yl)-5-(3-carboxymethoxyphenyl)-2-(4-sulfophenyl )-2H-tetrazolium) performed in HEK-293T cells (1×10⁵) treated for 24 hours at increasing doses of the composition of the invention (from 0.1 to 10x). The HEK-293T cells treated with a 'vehicle' solution (). i.e. Sodium Chloride [NaCl] at 0.8%) were used as a negative control of the experiment. The absorbance values measured at 490 nm are shown as 'fold' on cells treated with the 'vehicle' solution the mean ± standard deviation (SD) for the composition of the invention in HEK-293T cells is 7.1023x (regression equation: y = -0.0838x + 1.1938; R² = 0.9287). The regression equation, R2 and inhibitory concentration IC50 values are plotted. The IC50 was calculated by non-linear regression analysis {[inhibitor] versus response (three parameters)} with 'Quest Graph^{™} IC50. Calculator' (AAT Bioquest, Inc., January 8, 2024; https://www.aatbio.com/tools/ic50-calculator. The graphs were generated with Microsoft Excel (version 16.82). N = 6 independent experiments per group. R2, coefficient of determination. Y axis: absorbance values measured at 490 nm shown as 'fold' on cells treated with the 'vehicle' solution; X axis: increasing doses of the composition object of the invention (from 0.1 to 10x).
   **(B)** Graph showing the Caspase 3 assay in HEK-293T cells (1.5×10⁵) treated with increasing doses of the composition object of the invention (from 0.1 to 20x) for 24 hours. Treatment of the same cells with 0.2 micromolar staurosporine or with 'vehicle' solution (CTR, Sodium Chloride [NaCl] 0.8%) were used as positive and negative controls for the experiment, respectively. The relative fluorescent units (RFU) were recorded using a fluorescence reader with excitation at 380 nm and emission at 440 nm. Values represent the mean ± SD. **P < 0.01, ***P < 0.001 calculated by unpaired two-tailed Student's t test. The graphs were generated with Microsoft Excel (version 16.82). N = 9 independent experiments per group. NS, non-statistical. CTR, HEK-293T cells treated with the 'vehicle' solution (i.e. 0.8% Sodium Chloride [NaCl]). Y-axis: fluorescence unit (RFU) values with excitation at 380 nm and emission at 440 nm shown as 'fold' on cells treated with the 'vehicle' solution; X axis: increasing doses of the composition object of the invention (from 0.1 to 20x), Staurosporine, and Vehicle (CTR), as positive and negative controls, respectively, of the experiment.
   **(C)** The total ion chromatogram (TIC) of the composition object of the invention at 1x concentration obtained by UHPLC Q-Orbitrap HRMS is shown. The relative abundance of polyphenols is shown on the ordinate axis. The polyphenolic profile with the list of phenolic acids (benzoic and cinnamic acids) and flavonoids (flavonols, flavones, flavanones, isoflavones) are reported in Table 1 as µg/g values. Y-axis: Relative abundance; X axis: time (minutes, min). **(D)** Representative immunoblotting analysis (with antibodies against the indicated proteins: p65-NF-kB phosphorylated at Serine 311, p65-NF-kB, and β-Actin) in HEK-293T cells (1 × 10⁶ cells) treated for 24 hours with the composition object of the invention (at a concentration of 0.01x) or with its individual components (i.e., 0.625% Propolis, 0.125% Long-chain sodium polyphosphate synthesized as described above, 1.25% Mullein and 1.25% thyme). HEK-293T cells treated for 24 hours with 'vehicle' solution (i.e. 0.8% Sodium Chloride [NaCl]) were used as a negative control of the experiment. The numerical values represent the densitometric analysis of the intensities of the bands of p65-NF-kB phosphorylated in Serine 311 normalized on β-Actin in 2 independent experiments. The data show a reduction of p65-NF-kB phosphorylated in Serine 311 by 70% in HEK-293T cells treated with the composition object of the invention (at a concentration of 0.01x), by 30% in HEK-293T cells treated with Propolis extract (0.625% Propolis), by 60% in HEK-293T cells treated with long-chain sodium polyphosphate synthesized as described above (0.125%), 30% in HEK-293T cells treated with Mullein extract (1.25%) and 20% in HEK-293T cells treated with Thyme extract (1.25%). CTR, HEK-293T cells treated with the 'vehicle' solution (i.e. 0.8% Sodium Chloride [NaCl]).
**Figure 3: (A)** 'Heat Map' plot of bidirectional hierarchical clustering (Euclidean method, Complete Linkage) using the Z-Score for the normalized value (log2) representing the 'similarity' of gene expression levels between samples, showing n. 1210 differentially expressed genes (`Fold Change' of 2 and p-value <0.05). These data were obtained from RNA sequencing (RNAseq) analysis performed in HEK-293T cells treated with the composition of the invention at 0.01x concentration or with the vehicle (i.e. 0.8% Sodium Chloride [NaCl]) for 24 hours. N = 3 independent experiments. CTR, HEK-293T cells treated with the 'vehicle' solution (i.e. 0.8% Sodium Chloride [NaCl]). **(B)** Bubble plot graph showing the results of the 'Kyoto Encyclopedia of Genes and Genomes' (KEGG) analysis obtained using the differentially expressed genes ('Fold Change' of 2 and p-value <0.05) from the analysis of RNA sequencing (RNAseq) performed in HEK-293T cells treated with the composition of the invention at a concentration of 0.01x or with the vehicle (i.e. 0.8% Sodium Chloride [NaCl]) for 24 hours. The signaling pathways involved in inflammation or pathologies caused by microbial infection are shown in the graph. The statistical values of 'p-value' (-log10) are represented by the grey/black scale shown in the figure, while the number of differentially expressed genes (between the samples treated with the composition object of the invention compared to those of the cells treated with saline solution as control vehicle) belonging to the indicated signaling pathways are represented by the size of the 'bubbles'. N = 3 independent experiments per group.
   The plot was generated with SRplot (http://www.bioinformatics.com.cn/plot_basic_gopathway_enrichment_bubbleplot_081_en).
   **(C)** Analysis of the mRNA expression of the indicated genes (IL-10, IFNγ, and TNFα) normalized to the expression value of beta-actin (ACTB) used as a 'housekeeping' gene. The data are represented as variations in the expression levels of the indicated genes in the cells treated with the composition of the invention compared to those of the cells treated with the saline solution as a control vehicle (using the 2-ΔΔCt method). Real-time quantitative PCR (qPCR) analysis was carried out with SYBR-Green on RNA isolated from HEK-293T cells treated with the composition object of the invention at a concentration of 0.01x or with the vehicle (i.e. 0.8 % Sodium Chloride [NaCl]) for 24 hours. The data show that the composition object of the invention promotes the expression of IL-10, TNFα and IFNγ genes in HEK-293T cells. Data are represented as means ± standard deviation (SD); *P-value <0.05; **P-value <0.01 calculated by unpaired two-tailed Student's t-test. N = 3 independent experiments per group. The graphs were generated with Microsoft Excel (version 16.82). NS, non-statistical. CTR, HEK-293T cells treated with the 'vehicle' solution (i.e. 0.8% Sodium Chloride [NaCl]). Y axis: 2^-ΔΔCt values (fold on control, CTR) X axis : gene names in the qPCR analyses. **(D)** Graph representing the concentration of the peptides and cytokines indicated in the figure (pg/ml or ng/ml) measured by enzyme-linked immunosorbent assays (ELISA) carried out on the supernatants obtained from Caco-2 cells treated with the composition covered by the invention at a concentration of 0.01x, either with the vehicle (i.e. 0.8% Sodium Chloride [NaCl]) or on untreated cells after 24 hours. The data show that the composition object of the invention promotes the release of IL-10, HBD-2 and LL-37 in Caco-2 cells. Values are represented as mean ± standard deviation (SD). *P-value <0.05, **P-value <0.01, ***P-value <0.001 versus vehicle-treated cells calculated by unpaired two-tailed Student's t test; #P-value <0.05, ##P-value <0.01 compared to untreated cells calculated by unpaired two-tailed Student's t-test. Absorbance values were measured at 450 nm. Graphs were generated with Graph Pad Prism. N = 4 independent experiments per group. NT, Untreated cells. CTR, Caco-2 cells treated with the 'vehicle' solution (i.e. 0.8% Sodium Chloride [NaCl]). Y axis: ng/ml (IL-10, IFNγ, TNFα, LL-37) or pg/ml (HBD-2). X axis : gene names in the ELISA analyses.
**Figure 4: (A)** The experimental plan is shown in the figure. The Caco-2 cells were plated (5×10⁵ cells) and treated with the composition of the invention (therefore complete with long-chain sodium polyphosphate c, Mullein extract, Thyme extract, Propolis extract, at a concentration of 0.01x) or with a mixture containing only Mullein extract, Thyme extract, Propolis extract, therefore without long chain sodium polyphosphate (at a concentration of 0.01x). After 1 hour, cells were infected with SARS-CoV-2 viral particles belonging to VOC Omicron (EG.5) at a dose of 3 MOI. After 48 hours, the cells were lysed and their RNA was extracted. Cells treated with a 'vehicle' solution (i.e. 0.8% Sodium Chloride [NaCl]) and uninfected cells were used as negative controls for treatment and infection, respectively. qPCR, real time quantitative PCR. CTR, Caco-2 cells treated with the 'vehicle' solution (i.e. 0.8% Sodium Chloride [NaCl]).
   **(B)** Analysis of the expression of SARS-CoV-2 viral RNA corresponding to the viral genes sgN (subgenomic nucleoprotein RNA [marker of 'active' viral load], ORF1AB (ORF1A and ORF1B polyproteins), and E (envelope) normalized to the value of expression of the RNAseP gene used as a 'housekeeping' gene. The data are represented as variations in the expression levels of the indicated genes in the cells treated with the composition object of the invention (therefore complete with long-chain sodium polyphosphate consisting of 40 to 160 phosphate units, Mullein extract, Thyme extract, Propolis extract) and a mixture containing only Mullein extract, Thyme extract, Propolis extract (therefore without sodium polyphosphate long chain) compared to those of cells treated with saline as a control vehicle (using the 2-ΔΔCt method). Real-time quantitative PCR (qPCR) analysis was performed with the TaqMan approach on RNA isolated from Caco-2 cells pre-treated for 1 hour with the composition object of the invention (therefore complete with long-chain sodium polyphosphate], Mullein extract, Thyme extract, Propolis extract, at a concentration of 0.01x) or with a mixture containing only Mullein extract, Thyme extract, Propolis extract, therefore without long chain sodium polyphosphate (at a concentration of 0.01x ), and then infected with SARS-CoV-2 viral particles belonging to VOC Omicron (EG.5) at a dose of 3 MOI for 48 hours. Data are represented as means ± standard deviation (SD); *P-value <0.05, **P-value <0.01, ***P-value <0.001 calculated by unpaired two-tailed Student's t-test. N = 3 independent experiments per group. The graphs were generated with Microsoft Excel (version 16.82). NI, uninfected cells. The data show greater efficacy in the inhibition of viral genes in cells infected with SARS-CoV-2 pre-treated with the composition of the invention (therefore complete with long-chain sodium polyphosphate consisting of 40 to 160 phosphate units [ preferably from 80 to 120 phosphate units], Mullein extract, Thyme extract, Propolis extract), compared to those pre-treated with a mixture containing only extracts of Mullein, Thyme, and Propolis (therefore without long chain sodium polyphosphate ). CTR, HEK-293T cells treated with the 'vehicle' solution (i.e. 0.8% Sodium Chloride [NaCl]). N.I., uninfected cells. TPV, mixture of Thyme, Propolis and Mullein (without polyphosphates). Y axis: values of 2^-ΔΔCt (fold on control, CTR); X axis : gene names in the qPCR analyses.
   **(C)** 'Heat Map' plot of bidirectional hierarchical clustering (Euclidean method, Complete Linkage) using the Z-Score for the normalized value (log2) representing the 'similarity' of gene expression levels between samples, showing n. 5353 differentially expressed genes (`Fold Change' of 2 and p-value <0.05). These data were obtained from RNA sequencing (RNAseq) analyzes performed in Caco-2 cells pre-treated with the composition of the invention at 0.01x concentration or with the vehicle (i.e. 0.8% Sodium Chloride [ NaCl]) and infected with SARS-CoV-2 for 48 hours. N = 2 independent experiments. CTR, Caco-2 cells treated with the 'vehicle' solution (i.e. 0.8% Sodium Chloride [NaCl]). (D) Bubble plot showing the results of the 'Kyoto Encyclopedia of Genes and Genomes' (KEGG) analysis obtained using the differentially expressed genes ('Fold Change' of 2 and p-value <0.05) from the analyzes RNA sequencing (RNAseq) performed in Caco-2 cells pre-treated with the composition of the invention at a 0.01x concentration or with the vehicle solution (i.e. 0.8% Sodium Chloride [NaCl]) for 48 hours. The signaling pathways involved in inflammation or pathologies caused by microbial infection are shown in the graph. The statistical values of 'p-value' (-log10) are represented by the gray scale shown in the figure, while the number of differentially expressed genes (between the samples treated with the composition object of the invention compared to those of the cells treated with the saline solution as control vehicle) belonging to the indicated signaling pathways are represented by the size of the 'bubbles'. N = 2 independent experiments per group. The plot was generated with SRplot (http://www.bioinformatics.com.cn/plot_basic_gopathway_enrichment_bubbleplot_081_en).
   **(E)** Analysis of mRNA expression of the indicated genes (IL-10, IFNγ, TNFα, CXCL1, IL-12, TNFSF10, C3, IL-6, IL-1β, CXCL10, CXCL12, CXCL8) normalized to the β expression value -Actin (ACTB) used as a 'housekeeping' gene. The data are represented as variations in the expression levels of the indicated genes in Caco-2 cells infected with SARS-CoV-2 pre-treated with the composition object of the invention compared to those of cells pre-treated with saline solution as control vehicle (using the 2-ΔΔCt method). Real-time quantitative PCR (qPCR) analysis was carried out with SYBR-Green on RNA isolated from Caco-2 cells infected with SARS-CoV-2 pre-treated with the composition object of the invention compared to those of the cells pre-treated with saline as vehicle control (i.e. 0.8% Sodium Chloride [NaCl]) for 48 hours. The data show that pre-treatment with the composition object of the invention of Caco-2 cells infected with SARS-CoV-2 reduces the levels of TNFSF10, IL-1β, and CXCL10 (involved in hyper-inflammation and 'storm cytokines' and increases the expression levels of TNFα, CXCL1, IL-12, C3, and IL-6 (involved in the innate immune response) data are represented as means ± standard deviation (SD); .05; **P-value <0.01, ***P-value <0.001 calculated by unpaired Student's t-test = 3 independent experiments per group (version 16.82). NS, non-statistical, Caco-2 cells treated with the 'vehicle' solution (i.e. 0.8% Sodium Chloride [NaCl]) and infected with SARS-CoV-2 2^-ΔΔCt (fold on control, CTR). Y axis: values of 2^-ΔΔCt (fold on control, CTR) X axis: gene names in the qPCR analyses.
**Figure 5**: **(A)** The experimental plan is shown in the figure. Vero E6 cells (African green monkey kidney epithelial cells) were plated (5×10⁵ cells) and treated with the composition of the invention at 0.01x concentration or with the vehicle (i.e. 0.8% Sodium Chloride [NaCl]). After 1 hour, cells were infected with viral particles belonging to respiratory syncytial virus A (RSV-A) at a concentration of 0.2 multiplicity of infection (MOI). After 72 h, cells were lysed or fixed for RNA extraction or immunofluorescence analyses, respectively. Uninfected cells were used as a negative control for infection. qPCR, real time quantitative PCR. IF, immunofluorescence analysis. CTR, Vero E6 cells treated with the 'vehicle' solution (i.e. 0.8% Sodium Chloride [NaCl]).
   **(B)** Analysis of the expression of RSV viral RNA corresponding to the viral genes L (RNA-dependent viral RNA polymerase) and M (matrix) normalized to the expression value of the RNAseP gene used as a 'housekeeping' gene. The data are represented as variations in the expression levels of the indicated genes in the cells treated with the composition of the invention compared to those of the cells treated with the saline solution as a control vehicle (using the 2-ΔΔCt method). The real-time quantitative PCR (qPCR) analysis was carried out with a TaqMan approach on RNA isolated from Vero E6 cells (African green monkey kidney epithelial cells) pre-treated for 1 hour with the composition object of the invention at the concentration 0.01x or with vehicle (i.e. 0.8% Sodium Chloride [NaCl]), and then infected with viral particles belonging to respiratory syncytial virus A (RSV-A) at a concentration of 0.2 'multiplicity of infection' (MOI) for 72 hours. The data show that the composition object of the invention is effective in inhibiting the L and M viral genes in cells infected by RSV Data are represented as means ± standard deviation (SD); *P-value <0.05 calculated by unpaired two-tailed Student's t-test. N = 3 independent experiments per group. The graphs were generated with Microsoft Excel (version 16.82). NS, non-statistical. NI, uninfected cells. CTR, Vero E6 cells treated with the 'vehicle' solution (i.e. 0.8% Sodium Chloride [NaCl]) and infected with RSV-A. Y axis: 2^-ΔΔCt values (fold on control, CTR). X axis: gene names in the qPCR analyses.
   **(C)** Representative image of immunofluorescence (IF) carried out on fixed Vero E6 cells (African green monkey renal epithelial cells) pre-treated for 1 hour with the composition object of the invention at a concentration of 0.01x or with the vehicle (i.e. 0.8 % Sodium Chloride [NaCl]), and then infected with viral particles belonging to respiratory syncytial virus A (RSV-A) at a concentration of 0.2 'multiplicity of infection' (MOI) for 72 hours. An antibody against ACE2 was used. 4',6-diamidin-2-phenylindole (DAPI) to stain nuclei. Panels a-e, uninfected Vero E6 cells; Panels f-l, RSV-A-infected Vero E6 cells pre-treated with vehicle Sodium Chloride [NaCl] (0.8%) solution; m-q panels, Vero E6 cells infected with RSV-A pre-treated with the composition of the invention at a concentration of 0.01x. Images were acquired with the Elyra 7 microscope, and SIM2 images (panels f, l, q) were processed with Zeiss ZEN software (blue edition). Magnification, ×40 (a-c, f-h, m-o) ×63 (d-e, i-l, p-q). `Scale bar', 5 µm. More than 80 nuclei were counted. CTR, Vero E6 cells treated with the 'vehicle' solution (i.e. 0.8% Sodium Chloride [NaCl]) and infected with RSV-A.
   **(D)** 'Violin plot' graph showing respectively the percentage of syncytia identified in Vero E6 cells (African green monkey renal epithelial cells) pre-treated for 1 hour with the composition object of the invention at a concentration of 0.01x or with the vehicle (i.e. 0.8% Sodium Chloride [NaCl]), and then infected with viral particles belonging to respiratory syncytial virus A (RSV-A) at a concentration of 0.2 'multiplicity of infection' (MOI) for 72 hours. Uninfected cells were used as a negative control of the experiment. Quantification of syncytia percentages was performed by counting >80 cells for each condition. The P value was calculated by unpaired two-tailed Student's t test. N = 3 independent experiments per group. Plots were generated with SRplot (https://www.bioinformatics.com.cn/plot_basic_ggviolin_plot_113_en). CTR, Vero E6 cells treated with the 'vehicle' solution (i.e. 0.8% Sodium Chloride [NaCl]) and infected with RSV-A. Y axis: percentage of syncytia. X axis: experimental group names and conditions.
   **(E)** 'Violin plot' graph showing respectively the number of nuclei present in each syncytium identified in Vero E6 cells (African green monkey renal epithelial cells) pre-treated for 1 hour with the composition object of the invention at a concentration of 0.01x or with the vehicle (i.e. 0.8% Sodium Chloride [NaCl]), and then infected with viral particles belonging to respiratory syncytial virus A (RSV-A) at a concentration of 0.2 'multiplicity of infection' (MOI) for 72 hours. Uninfected cells were used as a negative control of the experiment. Quantification of syncytia and the number of nuclei per syncytium was performed by counting >80 cells for each condition. The P value was calculated by unpaired two-tailed Student's t test. N = 3 independent experiments per group. Plots were generated with SRplot (https://www.bioinformatics.com.cn/plot_basic_ggviolin_plot_113_en). CTR, Vero E6 cells treated with the 'vehicle' solution (i.e. 0.8% Sodium Chloride [NaCl]) and infected with RSV-A. Y axis: ratio of number of nuclei per syncytium (i.e. nuclei/syncytium). X axis: experimental group names and conditions.
**Figure 6: (A)** The experimental plan is shown in the figure. MDCK cells (isolated from normal kidney tissue of a cocker spaniel) were plated (1×10⁵ cells) and treated with the composition of the invention at 0.01x concentration or with the vehicle (i.e. 0.8% Sodium Chloride [NaCl ]). After 1 hour, the cells were infected with viral particles belonging to Influenza A (FLU-A) at a concentration of 3 'multiplicity of infection', MOI). After 6 h, cells were lysed for RNA extraction. Uninfected cells were used as a negative control for infection. qPCR, real time quantitative PCR. CTR, MDCK cells treated with the 'vehicle' solution (i.e. 0.8% Sodium Chloride [NaCl]).
   **(B)** Analysis of the expression of the viral RNA of FLU-A corresponding to the viral genes HA (hemagglutinin) and M (matrix) normalized to the expression value of the β-Actin gene (ACTB) used as a 'housekeeping' gene. The data are represented as variations in the expression levels of the indicated genes in the cells treated with the composition of the invention compared to those of the cells treated with the saline solution as a control vehicle (using the 2-ΔΔCt method). The real-time quantitative PCR (qPCR) analysis was carried out with a TaqMan approach on RNA isolated from MDCK (isolated from normal kidney tissue of a cocker spaniel) pre-treated for 1 hour with the composition object of the invention at the concentration 0.01x or with vehicle (i.e. 0.8% Sodium Chloride [NaCl]), and then infected with viral particles belonging to the Influenza A virus (FLU-A) at a concentration of 3 'multiplicity of infection' (MOI) for 6 hours. The data show that the composition object of the invention is effective in inhibiting the HA and M viral genes in cells infected with FLU-A. Data are represented as means ± standard deviation (SD); *P-value <0.05, **P-value <0.01 calculated by unpaired two-tailed Student's t-test. N = 3 independent experiments per group. The graphs were generated with Microsoft Excel (version 16.82). NI, uninfected cells. CTR, MDCK cells treated with the 'vehicle' solution (i.e. 0.8% Sodium Chloride [NaCl]) and infected with FLU-A. Y axis: 2^-ΔΔCt values (fold on control, CTR). X axis: gene names in the qPCR analyses.

### Description of the Tables

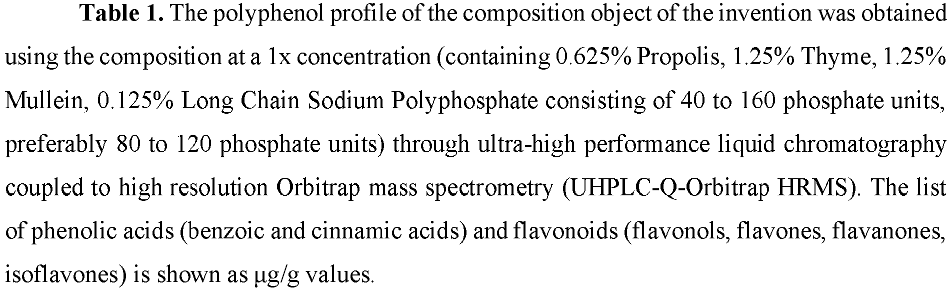

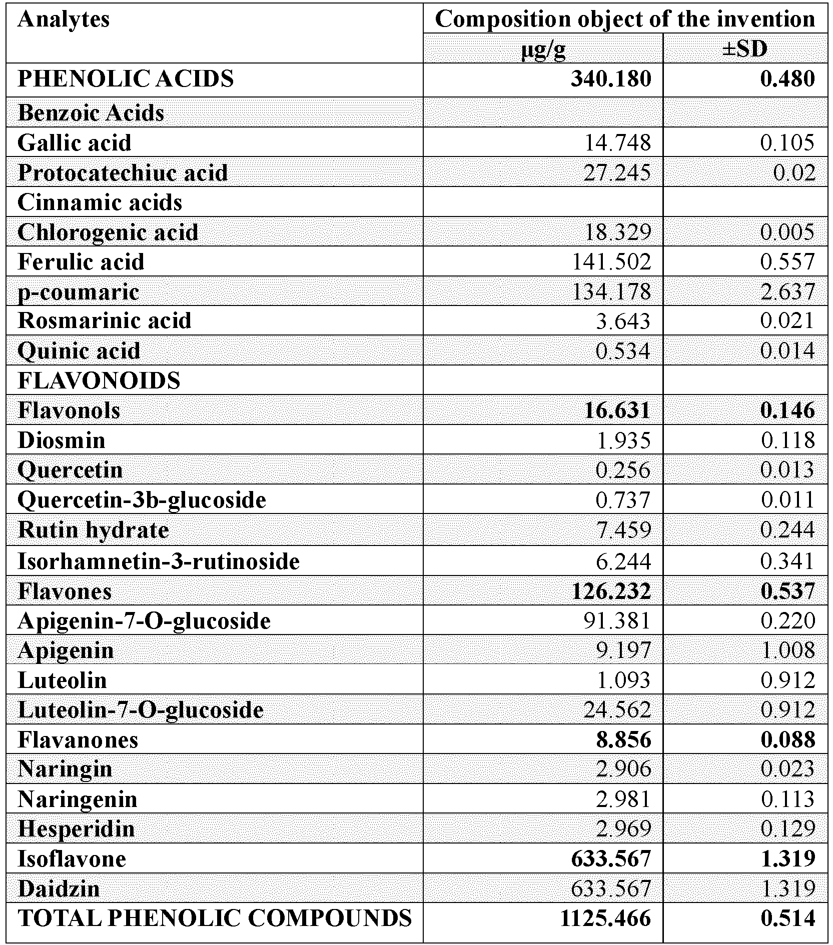

**Table 2. The polyphenol profile of aqueous extracts from Thyme and Mullein with Naviglio extractor through ultra-high performance liquid chromatography coupled to high resolution Orbitrap mass spectrometry (UHPLC-Q-Orbitrap HRMS). The list of phenolic acids (benzoic and cinnamic acids) and flavonoids (flavonols, flavones, flavanones, isoflavones) is shown as µg/g values.**

| | | | | |
|---|---|---|---|---|
| | | | | |

| **Analytes** | **Thime Aqueous extract** | | **Mullein Aqueous extract** | |
|---|---|---|---|---|
| | **µg/g** | **±SD** | **µg/g** | **±SD** |
| **PHENOLIC ACIDS** | | | | |
| **Benzoic Acids** | | | | |
| Gallic acid | 9.769 | 0.043 | 2.744 | 0.056 |
| Protocatechiuc acid | 15.926 | 0.337 | 2.372 | 0.018 |

| **Cinnamic acids** | | | | |
|---|---|---|---|---|
| Chlorogenic acid | 99.442 | 0.436 | 5.135 | 0.058 |
| Ferulic acid | 20.124 | 0.615 | 21.966 | 0.301 |
| p-coumaric | 1.004 | 0.061 | nf | nf |
| Rosmarinic acid | 241.737 | 0.015 | 0.341 | 0.075 |
| Quinic acid | 8.773 | 0.081 | 0.668 | 0.048 |
| SUM | 396.754 | 0.227 | 33.226 | 0.093 |

| **FLAVONOIDS** | | | | |
|---|---|---|---|---|
| **Flavonols** | | | | |
| Diosmin | 31.384 | 0.068 | 1.431 | 0.002 |
| Quercetin | 0.094 | 0.000 | nf | nf |
| Quercetin-3b-glucoside | 6.529 | 0.189 | nf | nf |
| Rutin hydrate | 4.022 | 0.035 | 32.373 | 0.314 |
| Isorhamnetin-3-rutinoside | 2.495 | 0.002 | 20.207 | 0.629 |
| SUM | 44.525 | 0.059 | 54.011 | 0.315 |

| **Flavones** | | | | |
|---|---|---|---|---|
| Apigenin-7-O-glucoside | 3.212 | 0.074 | nf | nf |
| Apigenin | 0.109 | 0.001 | 0.172 | 0.000 |
| Luteolin | 0.393 | 0.003 | 0.721 | 0.006 |
| Luteolin-7-O-glucoside | 72.527 | 1.318 | nf | nf |
| SUM | 83.24 | 0.349 | 0.892 | 0.003 |

| **Flavanones** | | | | |
|---|---|---|---|---|
| Naringin | 5.009 | 0.035 | 1.059 | 0.003 |
| Naringenin | 0.262 | 0.001 | nf | nf |
| Hesperidin | 60.472 | 0.011 | 1.131 | 0.093 |
| SUM | 65.744 | 0.015 | 2.19 | 0.048 |
| **Isoflavone** | | | | |
| Daidzin | nf | nf | nf | nf |
| SUM | nf | nf | nf | nf |
| **Total phenolic compounds** | 590.263 | 0.163 | 90.319 | 0.115 |

**Table 3. The bacteriostatic and bactericidal efficacy of the composition of the invention containing long-chain sodium polyphosphate consisting of 40 to 160 phosphate units, preferably 80 to 120 phosphate units, Propolis extract, Thyme extract, and extract of Mullein, used at increasing concentrations (from 0.5 to 20x) was tested against Gram-positive bacteria (Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucilaginosa, Rothia dentocariosa and Micrococcus luteus), Gram negatives (Escherichia coli, Pseudomonas aeruginosa, Acinetobacter lwoffii and Neisseria flavescens) and fungi (Candida albicans). The genus, Gram strain, strain name, origin, MIC and MBC values are shown. N=3 independent experiments. MIC, minimum inhibitory concentration; MBC, minimum bactericidal concentration.**

| | | | | | |
|---|---|---|---|---|---|
| | | | | | |

| | **Gram** | **Genere e Specie** | **Origine** | **MIC** | **MBC** |
|---|---|---|---|---|---|
| Bacteria | + | *Staphylococcus aureus* | ATCC 6538 | 0.40 | 0.4-1.2 |
| Bacteria | + | *Enterococcus hirae* | DSM 3320 | 4.00 | 4.00 |
| Bacteria | + | *Streptococcus mutans* | clinical strain | 0.40 | 0.8 |
| Bacteria | + | *Bacillus subtilis* | clinical strain | 0.40 | 0.40 |
| Bacteria | + | *Staphylococcus warneri* | clinical strain | 1.00 | 4-8 |
| Bacteria | + | *Streptococcus mitis* | clinical strain | 1.00 | 1.00 |
| Bacteria | + | *Streptococcus pneumoniae* | clinical strain | 1.00 | 1.00 |
| Bacteria | + | *Streptococcus pyogenes* | clinical strain | 1-2 | 1-2 |
| Bacteria | + | *Streptococcus salivarius* | clinical strain | 1.00 | 1-4 |
| Bacteria | + | *Rothia mucilaginosa* | clinical strain | 1.00 | 1-2 |
| Bacteria | + | *Rothia dentocariosa* | clinical strain | 1.00 | 1.00 |
| Bacteria | + | *Micrococcus luteus* | clinical strain | 1.00 | 1.00 |
| Bacteria | - | *Escherichia coli* | ATCC 13762 | 0.4-2 | 6.40 |
| Bacteria | - | *Pseudomonas aeruginosa* | ATCC 27853 | 1.00 | 4.00 |
| Bacteria | - | *Acinetobacter lwoffii* | clinical strain | 1.00 | 1.2 |
| Bacteria | - | *Neisseria, flavescens* | clinical strain | 1.00 | 1.00-2 |
| Fungi | | *Candida albicans* | clinical strain | 0.4 | 0.4-0.8 |

### Experimental part (Methods)

### Preparation and characterization of long-chain sodium polyphosphate consisting of 40 to 160 phosphate units, preferably 80 to 120 phosphate units

The long-chain polyphosphate was produced by a method based on the melting of monobasic sodium phosphate monohydrate H4NaO5P (≥ 99.0%, Carlo Erba, #480087) carried out at 700°C (250gr in porcelain/aluminium crucible; LLF, #Gndie/Ca1469, 300 ml capacity) using an electric oven (Oven L 40/11 with Controller B 510; Nabertherm.com). The time to reach 700°C is approximately 20 minutes (increase of 35°C/minute). Once 700°C is reached, the melting system operates at a constant temperature which is recorded for 1 hour. At the end of the fusion (approximately 220 ml of molten solution is obtained starting from 250g of monobasic sodium phosphate monohydrate H4NaO5P), the product is poured into a stainless steel mortar (Tera, dimensions 12 × 9.5 cm, capacity 350 ml) to achieve rapid 15 minute cooling to room temperature under sterile conditions. At the end of cooling, the solidified product (similar to silica) is frozen at -80°C for 3 hours and, subsequently, it is mechanically triturated using a homogenizer (MultiDrive IKA control Universal Milli-Voltage 220-240 V, #0025002643, IKA com) for 5 minutes, thus generating a powdered product. The resulting composition comes in the form of a powder and is dissolved in sterile isotonic saline (0.8% sodium chloride) in a sterile environment.

### Qualitative characterization of long-chain sodium polyphosphate consisting of 40 to 160 phosphate units, preferably 80 to 120 phosphate units

The length distribution of sodium polyphosphates is measured through electrophoresis. Acrylamide gel is prepared using 7 M urea, 15-20% acrylamide, methylene bisacrylamide 1:20 and TBE (Tris-HCl Borate, EDTA pH: 8.0). To this 0.0625% APS and 0.0625% v/v TEMED are added for gel polymerization. The dimensions of the gel are as follows: length 30 cm, width 14 cm and thickness 0.75 cm. Samples containing the generated Long Chain Sodium Polyphosphate are loaded onto gels using a loading 'buffer' (2x) containing: 0.2x TBE, 20% v/v glycerol, 0.1% bromophenol blue. Electrophoresis is performed at constant power (15 W) until bromophenol blue migrates up to approx. 12cm. The current must not exceed 25 mA. The TBE is used as a running buffer. Pre-electrophoresis (300 V, 1 hour) is performed to avoid poor band resolution due to excess salts. The staining of the gel is carried out with a solution consisting of 20% methanol, 1% glycerol, 0.1% o-toluidine blue for 15 minutes at room temperature. Gel decolorization is performed with a solution containing 20% v/v methanol, 1% v/v glycerol until the bands become visible [35].

### Preparation of the composition object of the invention

The pharmaceutical, nutraceutical, cosmetic or disinfectant composition object of the invention comprising long-chain sodium polyphosphate (consisting of 40 to 160 phosphate units, preferably 80 to 120 phosphate units), Mullein extract, Thyme extract, Propolis. The 1x concentration was obtained by dissolving 0.125 grams (g) of long-chain sodium polyphosphate consisting of 40 to 160 phosphate units (produced as described above), 0.625 g of dry extract of Propolis, 1.25 g of dry extract of thymus vulgaris leaves or 'thyme' or Thyme, 1.25 g of dry extract of verbascum thapsus flowers or 'mullein' or Mullein, and 0.8 g of sodium chloride in 100 ml of sterile water.

### Cell culture

Vero E6 (C1008; ATCC-CRL-1586), Caco-2 (ATCC, Middlesex, UK; accession number: HTB-37 L), MDCK (NBL-2; ATCC: CCL-34), HEK-293T cells and HEK-293T stable clones overexpressing human ACE2 (*i*.*e*., HEK-293T-ACE2) were grown in a humidified 37°C incubator with 5% CO2. The cells were cultured in feeder-free conditions using Dulbecco's modified Eagle's medium (41966-029; Gibco) with 10% fetal bovine serum (10270-106; Gibco), 2 mM L-glutamine (25030-024; Gibco), and 1% penicillin/streptomycin (P0781; Sigma-Aldrich), with medium changed daily. Cells were dissociated with Trypsin-EDTA solution (T4049, Sigma-Aldrich) when the culture reached ~80% confluency.

### In vitro treatment

Vero E6, HEK-293T, HEK293T-ACE2, Caco-2 and MDCK cells were plated in T25 flasks or 96 multiwells. The media were changed, and the cells were treated with polyPs (0.01 to 10%), propolis (0.5 to 5%), *Thymus vulgaris* L. leaves (0.1 to 5%), *Verbascum thapsus* L. (0.01 to 10%) or composition object of the invention (0.1 to 20x). After 24 hours of treatment, the cells were incubated with MTS assay, or lysed for proteins or RNA extraction. Vehicle-treated cells *(i.e.,* 0.8% NaCl) were used as the negative control for all the treatments.

### MTS assay and IC₅₀ evaluation

HEK-293T cells (1×10⁵) were plated in a 96-well plate and, the day after, they were treated with increasing concentrations of polyPs (0.01 to 10%), propolis (0.05 to 5%), *Thymus vulgaris* L. leaves (0.1 to 5%), *Verbascum thapsus* L. (0.01 to 10%) or composition object of the invention (0.1 to 10x) for 24 hours. Vehicle-treated cells *(i.e.,* 0.8% NaCl) were used as negative control. The cells were treated with MTS reagent (MTS Assay Kit, ab197010, Abcam) by directly adding it to cell culture media and incubated at 37°C for 1 hour in a humidified 37°C incubator with 5% CO2. The absorbance was measured at 490 nm using a multimode plate reader (PerkinElmer). The absorbance values and the related folds on vehicle-treated cells are shown in Additional file 1: Table S1. The IC₅₀ for polyPs, propolis, *Thymus vulgaris* L., *Verbascum thapsus* L. and composition object of the invention was calculated through nonlinear regression analysis {[inhibitor] versus response (three parameters)} with 'Quest Graph^{™} IC50 Calculator' (*AAT Bioquest, Inc.,* 8 Jan. 2024; https://www.aatbio.com/tools/ic50-calculator. *N* = 6 independent experiments per group.

### Caspase 3 assay

HEK-293T cells (1.5×10⁵) were plated in a 6-well plate and, the day after, they were treated with increasing concentrations of composition object of the invention (0.1 to 20x). Vehicle- *(i.e.,* 0.8% NaCl) and staurosporine- (0.2 µM) treated cells were used as negative and positive control, respectively. After 24 hours, the cells were washed, lysed in 200 ml/well of cell lysis buffer (PathScan^{®} Sandwich ELISA Lysis Buffer (1X) #7018, Cell Signaling Technology) left on ice for 5 min, and then stored at -80°C for 48 hours. Then, the cell lysates were diluted in 1x assay buffer complemented with 5 µM DTT to a final concentration of 4 mg/ml. Later, 100 µg protein lysates (25 ml) were mixed to 200 ml of 1x assay buffer complemented with 5 µM DTT and Ac-DEVD-AMC (1:40 dilution) in a black 96-well plate. The plate was then incubated at 37°C in the dark. The relative fluorescence units (RFU) were measured on a fluorescence plate reader with excitation at 380 nm and emission at 440 nm, using a multimode plate reader (PerkinElmer).

### Protein extraction and Immunoblotting

HEK-293T cells were lysed in 20 mM sodium phosphate, pH 7.4, 150 mM NaCl, 10% (v/v) glycerol, 1% (w/v) sodium deoxycholate, 1% (v/v) Triton X-100, supplemented with protease inhibitors (Roche). The cell lysates were cleared by centrifugation at 16,200× g for 30 min at room temperature, and the supernatants were removed and assayed for protein concentrations with Protein Assay Dye Reagent (BioRad). The cell lysates (20 µg) were resolved on 10% SDS-PAGE gels. The proteins were transferred to PVDF membranes (Millipore). After 1 h in blocking solution with 5% (w/v) dry milk fat in Tris-buffered saline containing 0.02% [v/v] Tween-20, the PVDF membranes were incubated with the primary antibodies overnight at 4 °C: anti-ACE2 (1:1000; ab15348), anti-β-actin (1:10000; A5441; Sigma), anti-NFκB p65 [C-20] (1:500, sc-372, SantaCruz) and anti-NFkB p65 phospho S311 (1:200, sc-166748, SantaCruz). The membranes were then incubated with the required secondary antibodies for 1 h at room temperature: secondary mouse or rabbit horseradish-peroxidase-conjugated antibodies (7076S or 7074S, respectively; Cell Signaling.), diluted in 5% (w/v) milk in TBS-Tween. The protein bands were visualized by chemiluminescence detection (Pierce-Thermo Fisher Scientific Inc., IL, USA). Densitometry analysis was performed with the Imaged software. The peak areas of the bands were measured on the densitometry plots, and the relative proportions (%) were calculated. Then, the density areas of the peaks were normalized with those of the loading controls, and the ratios for the corresponding controls are presented as fold-changes.

### Immunofluorescence analyses

Uninfected or RSV-infected-Vero E6 cells (5×10⁴) previously treated with composition object of the invention (0.01x) or vehicle (0.8% NaCl) were fixed in 4% paraformaldehyde (PFA) in phosphate-buffered saline (PBS) for 30 min, washed three times with PBS, and permeabilized for 15 min with 0.1% Triton X-100 (215680010; Acros Organics) diluted in PBS. The cells were then blocked with Antibody Diluent Block (ARD1001EA, Perkin Elmer) for 1 h at room temperature (RT), and incubated overnight with the primary antibody anti-ACE2 (1:200; ab15348, Abcam) at 4 °C in a humidified chamber. The cells were washed in PBS, and incubated with anti-rabbit Alexa Fluor 546 (1:200, #A10040; ThermoFisher), as the secondary antibody. DNA was stained by incubation with 4',6-diamidino-2- phenylindole (DAPI 1:5000; no. 62254, Thermo Fisher Scientific) for 10 min at RT. The cells were washed and mounted with coverslips with 50% glycerol (G5150, Sigma-Aldrich). Microscopy images were obtained using the Elyra 7 platform (Zeiss) with the optical Lattice SIM² technology (with the ZEN software, Zeiss, black edition), using the 40x or 63x oil immersion objective.

### Assessment of innate peptide and cytokines production via ELISA

Caco-2 cells were stimulated with vehicle (0.8% NaCl) and composition object of the invention (0.01x concentration) for 24 hours. Untreated cells exposed to only medium were used as negative control. Afterward, the supernatants were harvested and used for cytokine assay. The concentrations of HBD-2, LL-37, IFNγ, TNFα and IL-10 in cell supernatant were measured using specific human ELISA assay kits (Elabscience Biotechnology Inc. Wuhan, Hubei for HBD-2, [E-EL-H0996], LL-37 [E-EL-H2438], IFNγ [E-EL-H0108] and IL-10 [EEL-H6154], and Abcam for TNFα [#ab181421]). The minimum detection concentrations were 62.5 pg/ml for HBD-2, 15.6 ng/ml for LL-37, 15.63 ng/ml for IFNγ, 15.63 ng/ml for TNFα and 1.56 ng/ml for IL-10. The ELISAs were conducted according to the manufacturer's recommendations.

### SARS-CoV-2 isolation

SARS-CoV-2 (VOC OMICRON, EG.5) viruses were isolated from nasopharyngeal swabs from patients sample as previously described [24]. Briefly, Vero E6 cells (1×10⁶) were trypsinized and resuspended in Dulbecco's modified Eagle's medium (41966-029; Gibco) with 2% FBS in T25 flask to which the clinical specimen (100 µl) was added. The inoculated cultures were grown in a humidified 37°C incubator with 5% CO2. Seven days after infection, when cytopathic effects were observed, the cell monolayers were scrapped with the back of a pipette tip, while the cell culture supernatant containing the viral particles was aliquoted and frozen at -80°C. Viral lysates were used for total nucleic acid extraction for confirmatory testing and sequencing. The viral titration was made by using TaqPath COVID-19 RT-PCR Kit (A48102; Applied Biosystem).

### RSV isolation

Human respiratory syncytial virus A2 (RSV-A2, ATCC VR-1540) was isolated from Vero E6 cells. Briefly, Vero E6 cells (1×10⁶) were resuspended in Dulbecco's modified Eagle's medium (41966-029; Gibco) with 2% FBS in T25 flask to which RSV-A2 was added. The inoculated cultures were grown in a humidified 37°C incubator with 5% CO2. Five days after infection, the cell culture supernatant was aliquoted and frozen at -80°C. The viral titration was made by Reed-Muench method; TCDI50/ml: 2.00×10³.

### FLU-A isolation

Influenza A (FLU-A, H1N1 STRAIN A/PR/8/34, ATCC: VR-95) was isolated from MDCK cells. Briefly, MDCK cells (5×10⁵) were resuspended in Dulbecco's modified Eagle's medium (41966-029; Gibco) with 2% FBS in T25 flask to which FLU-A was added. The inoculated cultures were grown in a humidified 37°C incubator with 5% CO2. After 3 hours, the media culture supernatant was changed and replaced with Dulbecco's modified Eagle's medium (41966-029; Gibco) with 2% FBS and Trypsin, TPCK Treated (1:25 dilution; #20233, Thermo Scientific). Five days after infection, the cell culture supernatant was aliquoted and frozen at -80°C. The viral titration was made by Reed-Muench method; TCDI50/ml: 1.51×10⁴.

### SARS-CoV-2 infection

Caco-2 and HEK-293T-ACE2 cells (5×10⁵) were treated with 0.01x composition object of the invention or with 0.01x composition object of the invention without polyPs. Vehicle treated cells (0.8% NaCl) were used as negative control of the treatments. After 1 hour, the cells were infected with SARS-CoV-2 viral particles belonging to VOC Omicron EG.5 (3 MOI) for further 48 hours. Uninfected cells were used as the negative infection control. The cells were lysed, and their proteins or RNA were extracted for immunoblotting and qPCR analyses. These experiments were performed in a BLS3 authorised laboratory.

### RSV-A infection

Vero E6 cells (5×10⁵ for qPCR or 5×10⁴ for IF analyses) were plated and treated with 0.01x composition object of the invention. Vehicle treated cells (0.8% NaCl) were used as negative control of the treatments. After 1 hour, the cells were infected with RSV-A2 (ATCC: VR-1540) for further 72 hours. Uninfected cells were used as the negative infection control. The cells were lysed of fixed for RNA extraction or Immunofluorescence (IF) analyses. These experiments were performed in a BLS3 authorised laboratory.

### FLU-A infection

MDCK cells (1×105) were plated and treated with 0.01x composition object of the invention. Vehicle treated cells (0.8% NaCl) were used as negative control of the treatments. After 1 hour, the cells were infected with FLU-A (H1N1 STRAIN A/PR/8/34, ATCC: VR-95) for further 6 hours. Uninfected cells were used as the negative infection control. The cells were lysed and their RNA were extracted for qPCR analyses. These experiments were performed in a BLS3 authorised laboratory.

### UHPLC-Q-Orbitrap HRMS Analysis

The quali-quantitative profiles of 1x composition object of the invention were performed by Ultra High-Pressure Liquid Chromatograph (UHPLC, Dionex UltiMate 3000, Thermo Fisher Scientific, Waltham, MA, USA) equipped with a degassing system, a Quaternary UHPLC pump working at 1250 bar, and an autosampler device, as previously described ([29]). Chromatographic separation was carried out with a thermostat (T = 25_{°}C) Kinetex 1.7 µm F5 (50 × 2.1 mm, Phenomenex, Torrance, CA, USA) column. The mobile phase consisted of 0.1% formic acid (FA, purchased from Merck, Darmstadt, Germany) in water (A, deionized water [<18 MΩ x cm resistivity] obtained from a Milli-Q water purification system [Millipore, Bedford, MA, USA] and 0.1% FA in methanol (B, purchased from Merck, Darmstadt, Germany). The injection volume was 1 µL. The gradient elution program was as follows: an initial 0% B, increased to 40% B in 1 min, to 80% B in 1 min, and to 100% B in 3 min. The gradient was held for 4 min at 100% B, reduced to 0% B in 2 min, and another 2 min for column re-equilibration at 0%. The total run time was 13 min, and the flow rate was 0.5 mL/min. The mass spectrometer was operated in both negative and positive ion mode by setting 2 scan events: full ion MS and all ion fragmentation (AIF). The following settings were used in full MS mode: resolution power of 70,000 Full Width at Half Maximum (FWHM) (defined for m/z 200), scan range 80-1200 m/z, automatic gain control (AGC) target 1 × 106, injection time set to 200 ms and scan rate set at 2 scan/s. The ion source parameters were as follows: spray voltage 3.5 kV; capillary temperature 320 _{°}C; S-lens RF level 60, sheath gas pressure 18, auxiliary gas 3, and auxiliary gas heater temperature 350 _{°}C. For the scan event of AIF, the parameters in the positive and negative mode were set as follows: mass resolving power = 17,500 FWHM; maximum injection time = 200 ms; scan time = 0.10 s; ACG target = 1 × 105; scan range = 80-120 m/z; isolation window to 5.0 m/z; and retention time to 30 s. The collision energy was varied in the range of 10 to 60 eV to obtain representative product ion spectra. For accurate mass measurement, identification and confirmation were performed at a mass tolerance of 5 ppm for the molecular ion and for both fragments. Data analysis and processing were performed using Xcalibur software, v. 3.1.66.10 (Xcalibur, Thermo Fisher Scientific, Waltham, MA, USA).

### Microorganisms and growth conditions

The antimicrobial activity of composition object of the invention was evaluated against *Staphylococcus aureus* ATCC 6538 (American Type Culture Collection, Manassas, VA), *Pseudomonas aeruginosa* ATCC 27853, *Enterococcus hirae* DSM 3320 (Leibniz Institute DSMZ-German Collection of Microorganisms and Cell Cultures GmbH), *Escherichia coli* ATCC 13762, and against clinical strains of *Streptococcus mutans, Bacillus subtilis, Candida albicans, Streptococcus pyogenes, Streptococcus salivarius, Staphylococcus warneri, Streptococcus mitis, Streptococcus pneumoniae, Rothia mucilaginosa, Rothia dentocariosa, Micrococcus luteus, Neisseria flavescens* and *Acinetobacter lwoffii.* The identification of clinical isolates was performed by mass spectrometry using the Matrix Assisted Laser Desorption/Ionization (MALDI) mass spectrometer (Bruker Daltonics, MALDI Biotyper, Fremont, CA, USA), a high-throughput proteomic technique for identification of a variety of bacterial and fungal species, and biochemical phenotyping method in an BD Phoenix Automated Microbiology System (Becton Dickinson, BD Franklin Lakes, NJ, USA), according to the manufacturer's instruction. Bacteria were cultured aerobically in broth and agar media at 37°C. The media used were Tryptic soy (TS) (Oxoid, S.p.a., Rodano, Milano, Italy), Brain Heart Infusion (BHI) (Oxoid, S.p.a., Rodano, Milano, Italy) and Columbia CNA with 5% Sheep Blood with Colistin and Nalidixic Acid (Oxoid, S.p.a., Rodano, Milano, Italy). Microbial strains were maintained at 4°C on agar media. The isolates were stored frozen at -80°C in BHI broth supplemented with 10% glycerol (v/v) (Carlo Erba, Reagents, Milan, Italy) until use and the working cultures were activated in the respective broth at 37°C for 15-18 h.

### In Vitro Antibacterial Activity Assays

The susceptibility of reference and clinical strains to different concentrations of composition object of the invention was determined by dilution tube method of the Clinical and Laboratory Standards Institute using 1 × 10⁵ CFU/ml as standard inoculum (as described by Clinical and Laboratory Standards Institute [CLSI]; available at https://clsi.org/media/tc4b1paf/m10033_samplepages-1.pdf). The formulation was added in the multi-well achieving a ranging concentration from 0.5 to 20x and multi-well was incubated at 37°C for 24 h. After incubation, the optical density at A_{600 nm} was determined; subsequently an aliquot of each sample was spread into BHI or TS-agar plates and then incubated for 24/ 48 hours for the evaluation of viable counts. Minimum inhibitory concentration (MIC) value was assigned to the lowest concentration of composition object of the invention, which prevents bacterial growth. The minimum bactericidal concentration (MBC) was defined as the minimum extract concentration that killed 99% of bacteria in the initial inoculum.

### Transcriptomic analyses (RNA sequencing, RNAseq)

### RNA isolation and library construction and sequencing

Total RNA was isolated from HEK-293T and Caco-2 cells by using TRIzol RNA Isolation Reagent (#15596018; Ambion, Thermo Fisher Scientific) was quantified in a NanoDrop^{™} One/One^{C} Microvolume UV-Vis Spectrophotometer (Thermo Scientific^{™}), checked for purity and integrity and sequenced. Libraries were prepared using the TruSeq Stranded Total RNA LT Sample Prep Kit (Gold) according to the protocols recommended by the manufacturer. Trimmed reads are mapped to reference genome with HISAT2 (https://ccb.jhu.edu/software/hisat2/index.shtml), splice-aware aligner. After the read mapping, Stringtie (https://ccb.jhu.edu/software/stringtie/) was used for transcript assembly. Expression profile was calculated for each sample and transcript/gene as read count and FPKM (Fragment per Kilobase of transcript per Million mapped reads).

### Analysis of differentially expressed genes

DEG (Differentially Expressed Genes) analysis was performed on a comparison pair (composition object of the invention -treated- vs vehicle-treated cells). The read count value of known genes obtained through -e option of the StringTie was used as the original raw data. During data preprocessing, low quality transcripts are filtered. Afterwards, Trimmed Mean of M-values (TMM) Normalization were performed. Statistical analysis is performed using Fold Change, exactTest using edgeR per comparison pair. For significant lists, hierarchical clustering analysis (Euclidean Method, Complete Linkage) is performed to group the similar samples and genes. Pathway enrichment analysis was performed using KEGG database (http://www.genome.jp/kegg/). Graphs were generated by using SRplot (http://www.bioinformatics.com.cn/).

### RNA extraction and qPCR assays

Total RNA was isolated Caco-2, MDCK, HEK-293T and HEK-293T-ACE2 cells by using TRIzol RNA Isolation Reagent (#15596018; Ambion, Thermo Fisher Scientific), quantified in a NanoDrop^{™} One/One^{C} Microvolume UV-Vis Spectrophotometer (Thermo Scientific^{™}). The reverse transcription was performed with SuperScript^{™} VILO^{™} cDNA Synthesis Kit (Catalog number: 11754050, ThermoFisher scientific), according to the manufacturer instructions. The reverse transcription products (cDNA) were amplified by qRT-PCR using a PCR machine (2700; Applied Biosystems, Foster City, CA, USA). The cDNA preparation was through the cycling method by incubating the complete reaction mix as follows:
- cDNA reactions: [25 °C for 10 min and 50 °C for 10 min]
- Heat-inactivation: 85°C for 5 min
- Hold stage: 4 °C

### Human cytokines/ chemokines detection in HEK-293T and SARS-CoV-2-Tìinfected Caco-2 cells

The targets CXCL1, CXCL10, IFNγ, IL-10, IL-6, IL-12, TNFα, C3, TNFSF10 and ACTB in uninfected HEK-293T and Caco-2 cells and SARS-CoV-2-infected Caco-2 cells were detected with SYBR green approach by using BlastTaq 2x qPCR MasterMix (#4891; ABM). ACTB was used as the housekeeping gene used to normalize the quantification cycle (Cq) values of the other genes. These runs were performed on a PCR machine (QuantStudio^{™} 5 Real-Time PCR; Applied Biosystem) with the following thermal protocol:
- Enzyme Activation: 95°C for 3 min
- Denaturation Step: 95 °C for 15 sec
- Annealing/ extension (×40 cycles): 60 °C for 60 sec

The relative expression of the target genes was determined using the 2^{-ΔCt} method, as the fold increase compared with the controls. The data are presented as means ±SD of the 2^{-ΔCt} values (normalized to ACTB) of three replicates. The details of the primers used in these SYBR green assays are provided below:

| | | |
|---|---|---|
| - | ACTB Forward: GACCCAGATCATGTTTGAGACCTT | (SEQ ID NO:1) |
| - | ACTB Reverse: CCAGAGGCGTACAGGGATAGC | (SEQ ID NO:2) |
| - | IFNγ Forward: GAGTGTGGAGACCATCAAGGAAG | (SEQ ID NO:3) |
| - | IFNγ Reverse: TGCTTTGCGTTGGACATTCAAGTC | (SEQ ID NO:4) |
| - | IL-10 Forward: CCTGCCTAACATGCTTCGAGA | (SEQ ID NO:5) |
| - | IL-10 Reverse: TGTCCAGCTGATCCTTCATTTG | (SEQ ID NO:6) |
| - | TNFα Forward: TCTCTCTAATCAGCCCTCTGG | (SEQ ID NO:7) |
| - | TNFα Reverse: GCTACATGGGCTACAGGC | (SEQ ID NO:8) |

### sgN, E and ORFlab detection in SARS-CoV-2-infected-Caco-2 and -HEK-293T-ACE2 cells

The targets sgN, E and ORF1ab in SARS-CoV-2-infected Caco-2 and HEK-293T-ACE2 cells were detected with Taqman approach by using SARS-CoV-2 Viral3 kit (CE-IVD; #infect-004, BioMol laboratories; https://www.biomollaboratories.it/). These runs were performed using 75 ng (5 µL) RNA on a PCR machine (CFX96; BioRad; *in vitro* diagnostics IVD approved) under the following conditions:
- UNG incubation: 25 °C for 2 min
- Reverse transcription: 50 °C for 15 min
- Inactivation/denaturation: 95 °C for 3 min
- Denaturation and annealing (for 44 cycles): [95 °C for 3 s and 60 °C for 45 s].

The relative expression of the target genes was determined using the 2^{-ΔCt} method, as the fold increase compared with the controls. The data are presented as means ±SD of the 2^{-ΔΔCt} values (normalized to RNAseP) of three replicates. The details of the primers used in these assays are provided below:

| | |
|---|---|
| - sgN Forward: CAACCAACTTTTCGATCTCTTGTA | (SEQ ID NO:9) |
| - sgN Reverse: TCTGCTCCCTTCTGCGTAGA | (SEQ ID NO:10) |
| - Sonda sgN: 5'-FAM-ACTTCCTCAAGGAACAACATTGCCA-BBQ1-3' | (SEQ ID NO:11) |
| - Orflab Forward: CCCTGTGGTTTTACACCTTAA | (SEQ ID NO:12) |
| - Orflab Reverse: ACGATTGTGCATCAGCTGA | (SEQ ID NO:13) |
| - Sonda Orf1ab:5'-ROX- CCGTCTGCGGTATGTGGAAAGGTTATGG-BBQ2-3' | (SEQ ID NO:14) |
| - E Forward: ACAGGTACGTTAATAGTTAATAGCGT | (SEQ ID NO: 15) |
| - E Rovescio: ATATTGCAGCAGTACCACACA | (SEQ ID NO:16) |
| - Sonda E: 5' CYS-ACACTAGCCATCCTTACTGCGCTTCG BBQ2-3' | (SEQ ID NO: 17) |
| - RNAsi P Forward: ATGGCGGTGTTTGCAGATTT | (SEQ ID NO:18) |
| - RNAsi P Reverse: AGCAACAACTGAATAGCCA | (SEQ ID NO:19) |
| - Sonda RNAsi P: 5'-HEX-TTCTGACCTGAAGGCTCTGCGCG-BHQ1-3' | (SEQ ID NO:20) |

### L and M detection in RSV-infected Vero E6 cells

The targets L and M in RSV-infected Vero E6 cells were detected with Taqman approach by using Taqman Multiplex MasterMix kit (#4461882, Applied Biosystem). These runs were performed using a PCR machine (QuantStudio^{™} 5 Real-Time PCR, ThermoFisher Scientific) under the following conditions:
- Hold stage: 50°C for 2 min
- Denaturation Step: 95 °C for 10 min
- Denaturation and Annealing (×45 cycles): [95°C for 15 sec and 60 °C for 60 sec].
- Melt curve stage: [95°C for 15 sec, 60°C for 1 min and 95°C for 15 sec]

The relative expression of the target genes was determined using the 2^{-ΔCt} method, as the fold increase compared with the controls. The data are presented as means ±SD of the 2^{-ΔΔCt} values (normalized to RNAseP) of three replicates. The details of the primers used in these assays are provided below [30]:
- VIDRL Forward: AATACAGCCAAATCCAACCAACTTTACA (SEQ ID NO:21)
- VIDRL Reverse: GCCAAGGAAGCATGCAGTAAA (SEQ ID NO:22)
- VIDRL Probe: 5'- FAM-CTTTAGTGCACAATAGCA-BHQ1-3' (SEQ ID NO:23)
- RNAseP RPP30 Forward: ATG GCG GTG TTT GCA GAC TT (SEQ ID NO:24)
- RnaseP RPP30 Reverse: AGC AAC AAC TGA ATA GCC AAG G (SEQ ID NO:25)
- RNaseP RPP30 Probe: 5'- HEX- TTC TGA CCT GAA GGC TCT GCG CG-BHQ1-3' (SEQ ID NO:26)
- M RSV Forward: GGCAAATATGGAAACATACGTGAA (SEQ ID NO:27)
- M RSV Reverse: TCTTTTTCTAAGACATTGTATTGAACAG (SEQ ID NO:28)
- M RSV PROBE: 5'-FAM-AGCTTCACGAAGGCTCCACATACACAG-BHQ1-3' (SEQ ID NO:29)

### HA and M detection in FLU-A-infected MDCK cells

The targets HA and M in FLU-A-infected MDCK cells were detected with Taqman approach by using Taqman Multiplex MasterMix kit (#4461882, Applied Biosystem). These runs were performed using a PCR machine (QuantStudio^{™} 5 Real-Time PCR, ThermoFisher Scientific) under the following conditions:
- Hold stage: 50°C for 2 min
- Denaturation Step: 95 °C for 10 min
- Denaturation and Annealing (×45 cycles): [95°C for 15 sec and 60 °C for 60 sec].
- Melt curve stage: [95°C for 15 sec, 60°C for 1 min and 95°C for 15 sec]

The relative expression of the target genes was determined using the 2^{-ΔCt} method, as the fold increase compared with the controls. The data are presented as means ±SD of the 2^{-ΔΔCt} values (normalized to β-Actin [ACTB, Hs_01060665, ThermoFisher Scientific]) of three replicates. The details of the primers used in these assays are provided below (adapted from `WHO information for the molecular detection of influenza viruses', February 21, http://www.who.int/influenza/gisrs_laboratory/collaborating_centres/list/en/index.html):

| | |
|---|---|
| - H1N1 HA Forward: GACACAATAATATTTGAGGCAAATGG | (SEQ ID NO:30) |
| - H1N1 HA Reverse: GGGAGACTGCTGTTTATAGCTCC | (SEQ ID NO:31) |
| - SONDA H1N1 HA: 5'-FAMGCTTTCGCACTGAGTAGAGGC-BHQ1-3' | (SEQ ID NO:32) |
| - H1N1 M Forward: CTTCTAACCGAGGTCGAAACGTA | (SEQ ID NO:33) |
| - H1N1 M Reverse: GGTGACAGGATTGGTCTTGTCTTTA | (SEQ ID NO:34) |
| - SONDA H1N1 M: 5'-FAM-TCAGGCCCCCTCAAAGCCGAG-BHQ1 | (SEQ ID NO:35) |

### Statistical analysis

Statistical significance was defined as P < 0.05 by unpaired two-tailed Student's t tests. All of the data are given as means ± SD. In the figures, statistical significance is represented as follows: *P < 0.05, **P < 0.01, and ***P < 0.001. All of the data from the qPCR assays were analyzed using unpaired two-tailed Student's t tests, by comparing composition object of the invention -treated cells versus vehicle control-cells. ELISA data were analyzed by applying unpaired, two-tailed Student's t tests to the absorbance values by comparing composition object of the invention - treated cells versus vehicle control or untreated cells. The background values were subtracted from the absorbance values under each condition. Caspase-3 activities were analyzed by unpaired two-tailed Student's t tests by comparing RFU values measured after 1 hour of incubation. The background values were subtracted from the absorbance values under each condition. The IC50 for polyPs, propolis, Thymus vulgaris L., Verbascum thapsus L. and composition object of the invention was calculated through nonlinear regression analysis {[inhibitor] versus response (three parameters)} with 'Quest Graph^{™} IC50 Calculator' (AAT Bioquest, Inc., 8 Jan. 2024; https://www.aatbio.com/tools/ic50-calculator). Statistical analysis of RNAseq is performed using Fold Change, exactTest using edgeR per comparison pair. For significant lists, hierarchical clustering analysis (Euclidean Method, Complete Linkage) is performed to group the similar samples and genes. Pathway enrichment analysis was performed using KEGG database (http://www.genome.jp/kegg/). Graphs were generated by using Microsoft Excel (v.16.82), Graph Pad Prism and SRplot (http://www.bioinformatics.com.cn/).

### Bibliography

1. Patton, M.J., et al., COVID-19 bacteremic co-infection is a major risk factor for mortality, ICU admission, and mechanical ventilation. Crit Care, 2023. 27(1): p. 34.
2. Serigstad, S., et al., The changing spectrum of microbial aetiology of respiratory tract infections in hospitalized patients before and during the CO VID-19pandemic. BMC Infect Dis, 2022. 22(1): p. 763.
3. Gu, S., et al., Alterations of the Gut Microbiota in Patients With Coronavirus Disease 2019 or H1N1 Influenza. Clin Infect Dis, 2020. 71(10): p. 2669-2678.
4. Swets, M.C., et al., SARS-CoV-2 co-infection with influenza viruses, respiratory syncytial virus, or adenoviruses. Lancet, 2022. 399(10334): p. 1463-1464.
5. Chen, X., et al., The microbial coinfection in COVID-19. Appl Microbiol Biotechnol, 2020. 104(18): p. 7777-7785.
6. Kim, D., et al., Rates of Co-infection Between SARS-CoV-2 and Other Respiratory Pathogens. JAMA, 2020. 323(20): p. 2085-2086.
7. Chen, N., et al., Epidemiological and clinical characteristics of 99 cases of 2019 novel coronavirus pneumonia in Wuhan, China: a descriptive study. Lancet, 2020. 395(10223): p. 507-513.
8. Mehta, P., et al., COVID-19: consider cytokine storm syndromes and immunosuppression. Lancet, 2020. 395(10229): p. 1033-1034.
9. Zhou, F., et al., Clinical course and risk factors for mortality of adult inpatients with COVID-19 in Wuhan, China: a retrospective cohort study. Lancet, 2020. 395(10229): p. 1054-1062.
10. Yang, L., et al., COVID-19: immunopathogenesis and Immunotherapeutics. Signal Transduct Target Ther, 2020. 5(1): p. 128.
11. Boechat, J.L., et al., The immune response to SARS-CoV-2 and COVID-19 immunopathology - Current perspectives. Pulmonology, 2021. 27(5): p. 423-437.
12. Faraone, J.N., et al., Immune evasion and membrane fusion of SARS-CoV-2 XBB subvariants EG.5.1 and XBB.2.3. Emerg Microbes Infect, 2023. 12(2): p. 2270069.
13. Zhou, Q., et al., Use of non-steroidal anti-inflammatory drugs and adverse outcomes during the COVID-19 pandemic: A systematic review and meta-analysis. EClinicalMedicine, 2022. 46:p. 101373.
14. Wongrakpanich, S., et al., A Comprehensive Review of Non-Steroidal Anti-Inflammatory Drug Use in The Elderly. Aging Dis, 2018. 9(1): p. 143-150.
15. Yahfoufi, N., et al., The Immunomodulatory and Anti-Inflammatory Role of Polyphenols. Nutrients, 2018. 10(11).
16. Guo, Q., et al., NF-kappaB in biology and targeted therapy: new insights and translational implications. Signal Transduct Target Ther, 2024. 9(1): p. 53.
17. Zhu, L., et al., Propolis polyphenols: A review on the composition and anti-obesity mechanism of different types of propolis polyphenols. Front Nutr, 2023. 10: p. 1066789.
18. Taghouti, M., et al., Polyphenol composition and biological activity of Thymus citriodorus and Thymus vulgaris: Comparison with endemic Iberian Thymus species. Food Chem, 2020. 331: p. 127362.
19. Rajbhandari, M., et al., Antiviral activity of some plants used in Nepalese traditional medicine. Evid Based Complement Alternat Med, 2009. 6(4): p. 517-22.
20. Additives, E.P.o.F., et al., Re-evaluation of phosphoric acid-phosphates - di-, tri- and polyphosphates (E 338-341, E 343, E 450-452) as food additives and the safety of proposed extension of use. EFSA J, 2019. 17(6): p. e05674.
21. Muller, F., et al., Platelet polyphosphates are proinflammatory and procoagulant mediators in vivo. Cell, 2009. 139(6): p. 1143-56.
22. Müller, W.E.G., et al., The physiological polyphosphate as a healing biomaterial for chronic wounds: Crucial roles of its antibacterial and unique metabolic energy supplying properties. Journal of Materials Science & Technology, 2023. 135: p. 170-185.
23. Lorenz, B., et al., Anti-HIV-1 activity of inorganic polyphosphates. J Acquir Immune Defic Syndr Hum Retrovirol, 1997. 14(2): p. 110-8.
24. Ferrucci, V., et al., Long-chain polyphosphates impair SARS-CoV-2 infection and replication. Sci Signal, 2021. 14(690).
25. Neufurth, M., et al., The inorganic polymer, polyphosphate, blocks binding of SARS-CoV-2 spike protein to ACE2 receptor at physiological concentrations. Biochem Pharmacol, 2020. 182:p. 114215.
26. Muller, W.E.G., et al., An unexpected biomaterial against SARS-CoV-2: Bio-polyphosphate blocks binding of the viral spike to the cell receptor. Mater Today (Kidlington), 2021. 51: p. 504-524.
27. Schepler, H., et al., The therapeutic potential of inorganic polyphosphate: A versatile physiological polymer to control coronavirus disease (COVID-19). Theranostics, 2021. 11(13): p. 6193-6213.
28. Muller, W.E.G., et al., The biomaterial polyphosphate blocks stoichiometric binding of the SARS-CoV-2 S-protein to the cellular ACE2 receptor. Biomater Sci, 2020. 8(23): p. 6603-6610.
29. Izzo, L., et al., Colon Bioaccessibility under In Vitro Gastrointestinal Digestion of a Red Cabbage Extract Chemically Profiled through UHPLC-Q-Orbitrap HRMS. Antioxidants (Basel), 2020. 9(10).
30. Williams, T., et al., Results from the second WHO external quality assessment for the molecular detection of respiratory syncytial virus, 2019-2020. Influenza Other Respir Viruses, 2023. 17(1): p. e13073.

## Claims

1. A pharmaceutical, nutraceutical, cosmetic or disinfectant composition comprising: long-chain sodium polyphosphate consisting of 40 to 160, preferably 80 to 120 phosphate units; Mullein (Verbascum Thapsus) extract; Thyme (Thymus vulgaris) extract; Propolis extract.

2. Composition according to claim 1, comprising:
- 0.01% to 3%, preferably 0.05% to 2.5% of long-chain sodium polyphosphate
- from 0.1% to 5%, preferably from 0.2 to 2% of Mullein (Verbascum Thapsus) extract
- from 0.1% to 5%, preferably from 0.2% to 2% of Thyme (Thymus vulgaris) extract
- from 0.05% to 5%, preferably from 0.2% to 2% of Propolis extract
wherein said percentages refer to the total weight of the composition.

3. Composition according to claims 1 or 2, wherein said extracts of Propolis, Thyme and Mullein are water dispersible, dry, oily or sterol lipid extracts.

4. Composition according to claims 1-3, wherein said composition is in a form suitable for oral use, preferably in the form of a nanospray, aerosol, mouthwash or toothpaste; or in topical form, preferably in the form of cream, serum, cleanser, mousse, intimate cleanser, lip balm or lipstick; or in a form for systemic administration, preferably in the form of tablets, capsules, powders, granules, suspensions, syrups.

5. Composition according to claims 1-4 further containing excipients or pharmaceutical vehicles suitable for the form of administration, preferably diluents, binders, lubricants, disintegrating agents and/or colorants.

6. Composition according to claims 1-5, which is selected from the following:
(i) composition in a form suitable for oral administration, comprising:
- Propolis extract;
- Mullein (Verbascum Thapsus) extract;
- Thyme (Thymus vulgaris) extract;
- long chain sodium polyphosphate consisting of 40 to 160, preferably 80 to 120 phosphate units;
- sodium chloride;
- water;
and optionally one of more of Aloe vera (Aloe barbadensis) extract, Calendula officinalis L., capolini extract, Collagen, Echinacea (Echinacea purpurea (L.) Moench) extract, Erisimo (Sisymbrium officinalis) extract, Erisimo (Hedge mustard) extract, Honey, Hyaluronic Acid, Mint (Mentha Piperita L) extract, N-Acetyleysteine, Panthenol, Pinus sibirica extract, Plantain extract, polydecene, potassium sorbate, potassium phosphate dibasic, potassium phosphate monobasic, Rosmarinus officinalis cincoliferum extract, seawater, sodium benzoate, strawberry, Tocopheryl Acetate (Vitamin E), Xylitol.
(ii) composition in cream form comprising:
- Propolis extract
- Mullein (Verbascum Thapsus) extract
- Thyme (Thymus vulgaris) extract
- long chain sodium polyphosphate consisting of 40 to 160, preferably 80 to 120 phosphate units
- water
and one or more of
1,2 hexanediol, acetyl hexapeptide-37, allantoin, Aloe barbadensis extract, aminomethyl propanol, Arnica montana, beeswax, benzyl alcohol, Berberis vulgaris bark extract, betaine, bisabolol, Borago officinalis extract, butylene glycol, butylhydroxytoluene, Butyrospermum parkii butter (Karitè), Calendula officinalis extract, Cananga odorata flower oil (Ylang Ylang oil), candelilla wax, caprylyl glycol, carbomer (carbopol), cera alba, cetearyl alcohol, cetyl alcohol, cetyl palmitate, citic acid, Citrus aurantium amara flower oil, coco-caprylate, cocos nucifera fruit, coumarin, ethylhexylglycerin, Cucumis sativas (Cucumber) seed oil, dead sea salts, decyl oleate, diatomaceous earth, dicaprylyl carbonate, dimethicone, disodium EDTA, ethylhexyl stearate, ethylhexylglycerin, glycerin, glyceryl caprylate, glyceryl linolenate, glyceryl oleate, glyceryl stearate SE, Hamamelis virginiana leaf extract, Helianthus annuus seed oil, hydrogenated vegetable oil, Jojoba oil, lactic acid, lanolin, lecithin, linalool, Malva sylvestris extract, Olea europaea fruit oil, olive oil, ozokerite, panthenol, paraffinum liquidum, parfum, pentylene glycol, Persea gratisima oil, petrolatum, phenoxyethanol, polyethylene glycol, polyglyceryl-3 caprate, Polyglyceryl 6 distearate, polysorbate, potassium palmitoyl hydrolyzed wheat protein, potassium sorbate, Prunus amygdalus dulcis oil, Rosa canina (Rose hip) fruit oil, sodium benzoate, sodium gluconate, sodium hyaluronate, sodium hydroxide, sodium polyaerylate, sorbitol, steareth-2, steareth-21, stearyl alcohol, Styrax benzoin resin extract, sucrose polystearate, theobroma cacao butter, tocopheryl acetate, Triticum vulgare germ oil caprylic/capric triglyceride, Triticum vulgaris oil, urea, vitamin E, xanthan gum, zinc oxide
(iii) composition in the form of sun protection cream containing:
- Propolis extract
- Mullein (Verbascum Thapsus) extract
- Thyme (Thymus vulgaris) extract
- long chain sodium polyphosphate consisting of 40 to 160, preferably 80 to 120 phosphate units
- water
- one or more of collagen; an anti-wrinkle peptides which chelate metals and self-renew the skin selected from acetyl hexapeptide-37 and acetyl hexapeptide-8; alumina dehydroacetic acid, aloe barbadensis leaf juice, arnica (arnica montana), astaxanthin, benzyl alcohol, bht, butyrospermum parkii butter, butylene glycol, calendula officinalis flower extract, caprylic/capric triglyceride, caprylyl glycol, carthamus tinctorius seed oil, palmitoyl tripeptide 5, cera alba, cetearyl alcohol, cetyl alchol, chondrus crispus powder, ci 77891/titanium dioxide, citric acid, collagen, coumarin, diatomaceous earth, dicaprylyl carbonate, dimethicone, disodium edta, dodecane, ethylexyl stearate, ethylhexylglycerin, glyceryl caprylate, glyceryl linoleate, glyceryl linolenate, glyceryl oleate, hamamelis virginiana leaf extract, helianthus annuus seed oil, hyaluronic acid, lanolin, lecithin, linalool, ozokerite, panthenol, parfum, paraffinum liquidum, persea gratissima oil, phenoxyethanol, polyglyceryl-2-dipolydroxystearate, polyglyceryl-3 caprate, polyglyceryl-3 disostearate, potassium sorbate, propanediol, prunus amygdalus duleis oil, simmondsie chinensis seed oil, sodium benzoate, sodium hyaluronate, sodium polyacrylate, sorbitol, stearic acid, steareth-2, steareth-21, stearyl alcohol, tritium vulgare germ oil, xanthan gum, zinc oxide.

7. Pharmaceutical or nutraceutical composition according to claims 1-6, for use in the prevention or treatment of an infection caused by viruses, in particular by SARS-CoV-2 virus, respiratory syncytial virus RSV-A or RSV-B, Influenza (FLU-A, FLU-B, or FLU-C) and herpes simplex.

8. Composition for use according to claim 7, wherein said composition is administered in therapeutic association with a further pharmacological agent, in particular with an antiviral agent.

9. Pharmaceutical or nutraceutical composition according to claims 1-6, for use in the prevention or treatment of a bacterial or fungal infection, in cases of candidiasis, mucositis, pharyngitis, gingivitis, stomatitis, periodontitis and dental infections caused by Gram positive bacteria, in particular *Staphylococcus aureus, Enterococcus hirae, Streptococcus mutans, Bacillus subtilis, Staphylococcus warneri, Streptococcus mitis, Streptococcus pneumoniae, Streptococcus pyogenes, Streptococcus salivarius, Rothia mucillaginosa, Rothia dentocariosa and Micrococcus luteus;* Gram negative bacteria, in particular *Escherichia coli, Pseudomonas aeruginosa, Acinetobacter, lwoffii and Neisseria flavescens;* bacteria selected from: *Enterococcus faecium, Staphylococcus aureus, Klebsiella pneumoniae, Acinetobacter baumannii, Pseudomonas aeruginosa, and Enterobacter species;* or fungal pathogens, in particular *Candida albicans.*

10. Composition for use according to claim 9, wherein said composition is administered in therapeutic association with a further pharmacological agent, in particular with an antibiotic agent.

11. Pharmaceutical or nutraceutical composition according to claims 1-6, for use in the treatment or prevention of inflammation of the skin due to lesions of benign nature, in particular dermatitis including atopic dermatitis, contact dermatitis, seborrheic dermatitis, nummular dermatitis, stasis dermatitis, dyshidrotic dermatitis, neurodermatitis, perioral dermatitis; psoriasis including vulgaris, plaque, guttate, inverse, pustular, eritrodermic, nail, psoriatic arthritis; acne, rhagades, fissures, cracks, ulcers, sores, bedsores including pressure ulcers; or due to malignancy, in particular squamous cell carcinoma, cellular carcinoma basal or basal cell carcinoma, and melanoma.

12. Pharmaceutical or nutraceutical composition according to claims 1-6, for use in the prevention or treatment of oral candidiasis, mucositis, pharyngitis, gingivitis, stomatitis, periodontitis and dental infections caused by bacterial and fungal pathogens, in particular *Candida albicans,* in immunosuppressed subjects treated with radiotherapy and/or chemotherapy for head and neck tumors, and skin and breast tumors.

13. Composition for use according to claim 12, wherein said composition is administered in therapeutic association with a further pharmacological agent, in particular with an antibiotic or an antifungal agent.

14. Composition for use according to claims 6-13 in the prevention or treatment of bacterial, viral, fungal infection occurring in surgery or post-surgery intervention in human and veterinary applications.

15. Composition according to claims 1-7, or for use according to claims 6-13, wherein the long-chain sodium polyphosphate is obtainable by a process comprising:
(i) melting sodium monophosphate at a temperature from 300°C to 750°, preferably 700°C, for a period of from 1 hr to 3 hr, preferably 1.5 hr;
(ii) cooling the product of step (i) at room temperature for a period of 5 min to 30, preferably 15 min, thereby obtaining a solid product;
(iii) freezing the product of (ii) at a temperature from -20°C to -80°C, preferably -60°C for a period of from 1 hr to 24 hr, preferably 3 hrs;
and optionally
(iv) mechanically triturating the product of (iii) for a period of 5 to 30, preferably 15 minutes, thereby obtaining a product in powder form;
(v) dissolving the product of (iv) in water containing 0.7% to 3.5%, preferably 0.8% or 3% w/vol sodium chloride.
